# EUROPEAN PATENT APPLICATION

(11) **EP 1 832 298 A1**
(43) Date of publication of application: **12.09.2007**
(21) Application number: 05822550.9
(22) Date of filing: 27.12.2005
(51) Int. Cl.: A61K 47/32, A61K 9/14, A61K 9/22, A61K 9/54, A61K 31/13, A61K 31/445, A61K 47/10, A61K 47/26, A61K 47/38, A61P 25/28

(54) **MATRIX-TYPE CONTROLLED RELEASE PREPARATION COMPRISING BASIC SUBSTANCE OR SALT THEREOF, AND PROCESS FOR PRODUCTION OF THE SAME**

(30) Priority: 27.12.2004 JP 2004376770; 06.04.2005 JP 2005110404; 28.04.2005 JP 2005132338; 28.04.2005 US 675482 P
(71) Applicant: Eisai R&D Management Co., Ltd., Tokyo 112-8088 (JP)
(72) Inventor: UEKI, Yosuke, Kakamigahara-shi, Gifu 5016195 (JP); FUJIOKA, Satoshi, Kakamigahara-shi, Gifu 5016195 (JP)
(74) Representative: HOFFMANN EITLE
(86) International application number: PCT/JP2005/023853
(87) International publication number: WO 2006/070781

(57) **Abstract**

A matrix type sustained-release preparation and a manufacturing method therefor are provided wherein dissolution with low pH dependence of a basic drug or a salt thereof at the early stage of dissolution can be ensured in a dissolution test, and wherein as the dissolution test proceeds, a ratio of a dissolution rate of the basic drug or the salt thereof in an acidic test solution to a dissolution rate of the basic drug or the salt thereof in a neutral test solution (dissolution rate in the acidic test solution /dissolution rate in the neutral test solution) decreases with dissolution time at the late stage of dissolution, as compared to the early stage of dissolution. According to the present invention, the matrix type sustained-release preparation contains a basic drug or a salt thereof and at least one enteric polymer, in which solubility of the basic drug or the salt thereof in a 0.1 N hydrochloric acid solution and a neutral aqueous solution, pH 6.0 is higher than in a basic aqueous solution, pH 8.0.

## Description

### Technical Field

The present invention relates to a matrix type sustained-release preparation containing a basic drug or a salt thereof, more specifically, relates to the preparation which not only inhibits the initial drug burst (rapid drug release immediately after dissolution) in a dissolution test, but also ensures dissolution with low pH dependence of the basic drug or the salt thereof at the early stage of dissolution, in the dissolution test. The present invention also relates to a matrix type sustained-release preparation containing a basic drug or a salt thereof in which a ratio of a dissolution rate of the basic drug or the salt thereof in an acidic test solution to a dissolution rate of the basic drug or the salt thereof in a neutral test solution (dissolution rate in the acidic test solution / dissolution rate in the neutral test solution) decreases with dissolution time at the late stage of dissolution, as compared to the early stage of dissolution.

### Background Art

As compared to ordinary rapid-release preparations, a sustained-release preparation containing a physiologically active drug allows blood concentrations of the drug to be maintained for a long time at or above the effective therapeutic concentration. Accordingly, by achieving the sustained-release characteristics of a drug it is possible to reduce the number of administrations while providing the same or better therapeutic effects, potentially improving compliance. With the sustained-release characteristics of the drug, it is also possible to avoid a rapid increase in blood concentration of the drug immediately after administration, thus potentially reducing adverse effects, toxicity and the like due to the drug.

In general, the sustained-release preparation containing the drug which is physiologically active can be classified into two type preparations; (1) a sustained-release coated type preparation, in which release of the drug is controlled by coating a surface of a core particle or a core tablet containing a physiologically active drug with a sustained-release coating: (2) a matrix type preparation, in which the drug and a sustained-release base are distributed uniformly in the preparation.

Because drug release is affected by the uniformity of the coating in the sustained-release coated preparations, the coating conditions of the sustained-release coating have to be strictly controlled, and productivity is often low because of long coating times. Further, when applying the sustained-release coating to granules, the sustained-release coating is generally applied after the drug has been stacked on a core particle generally containing crystalline cellulose or sucrose. Consequently, a size of the preparation tends to be large when multiple layers of the sustained-release coating are applied or when a preparation contains a large amount of the drug, making it harder to administer orally.

On the other hand, the matrix type sustained-release preparations have a structure in which the drug and the sustained-release base are present uniformly in the preparation and do not require the same strict production conditions as the sustained-release coated preparations, and can be produced by manufacturing operations similar to those for the ordinary rapid-release preparations. Accordingly, high productivity can be expected for the matrix type sustained-release preparations. In addition, it is easy to prepare the matrix type sustained-release preparation even when it contains a large quantity of the drug, and a size of the preparation does not need to be large. Thus, the matrix type sustained-release preparations are more useful than the sustained-release coated preparations from the standpoint of productivity and smaller size of the preparations.

However, when the physiologically active drug is a basic drug or a salt thereof, the following problems generally occur when preparing a matrix type sustained-release preparation using a water-insoluble base:

The first problem is that in a dissolution test of a matrix type sustained-release preparation containing a basic drug or a salt thereof, the dissolution rate of the basic drug or the salt thereof with dissolution time is generally much lower in a basic test solution than in an acidic test solution. This is because solubility of the basic drug or the salt thereof in an aqueous solvent is lower in a neutral and an alkaline pH range than in an acidic pH range. Because the sustained-release preparations generally comprises a larger amount of the drug than that of the rapid-release preparations, if a retention time of the sustained-release preparation in stomach is prolonged, there are risks of an unexpected increase in blood concentration of the basic drug or the salt thereof and onset of the adverse effects involved. This risk of the onset of the adverse effects is particularly serious in the case of the basic drug or the salt thereof with strong adverse effects and the basic drug and the salt thereof with a narrow safety range of blood concentration.

The second problem is that the release rate of the basic drug or the salt thereof from the matrix type sustained-release preparation is lower at the late stage of dissolution than at the early stage of dissolution in the dissolution test. If the dissolution speed of the basic drug or the salt thereof from the sustained-release preparation in the acidic test solution is controlled with the aim of avoiding the first problem described above (that is, the rapid increase in blood concentration due to prolonged retention time of the drug or the salt thereof in stomach), in the case of the sustained-release preparation with a short gastric emptying time, this preparation is excreted with most of the drug remaining in the preparation, thus decreasing bioavailability and posing a different risk that pharmacologically effective concentrations will not be achieved.

This is because the water-insoluble matrix does not dissolve in the dissolution test solution, and consequently, the diffusion distance in the matrix that the drug must pass through in order to be released becomes longer with dissolution time. Thus, if the release speed of the basic drug or the salt thereof is inhibited too much at the early stage of dissolution, there may be a higher risk that the matrix type sustained-release preparation is excreted with most of the drug remaining in the preparation outside the body. Moreover, as the matrix type sustained-release preparation moves from the stomach to the small intestine, the drug release speed decreases as a pH surrounding the preparation becomes neutral or weakly alkaline, thus increasing the risk that this preparation is excreted with most of the drug remaining in the preparation outside the body. This is undesirable because it reduces the bioavailability of the drug and gives the uncertainty of the pharmacological effects.

The following related art documents pertain to the matrix type sustained-release preparations containing the basic drug or the salt thereof. U.S. Patent No. 4,792,452 discloses, for example, a matrix preparation which contains a basic drug or a salt thereof, alginic acid or a salt thereof, a pH-independent water-soluble gelatinizing agent and a binder. U.S. Patent No. 4,968,508 discloses, for example, a matrix preparation which contains Cefaclor, an acrylic acid polymer which dissolves at pH 5.0 to 7.4 and a hydrophilic polymer. Japanese Patent Application Laid-Open No. H6-199657 discloses, for example, that a pH-dependent dissolution of a drug with higher solubility in an acetic acid buffer, pH 4.0 than in 1 st fluid and 2nd fluid of the Japanese Pharmacopoeia can be improved by preparing matrix tablets containing a water-soluble polymer, "a carboxyvinyl polymer or methylvinyl ether-maleic anhydride copolymer" and an enteric base. U.S. Patent No. 6,287,599 discloses, for example, a matrix preparation which contains a basic drug or a salt thereof with pH-dependent solubility, a pH-independent sustained-release base and a pH-dependent additive such as an enteric base, an organic acid and the like (which increases the dissolution rate of the drug from a tablet at a pH in excess of 5.5).

However, these related art documents relate to the matrix type sustained-release preparations aimed only at pH-independent release of the drug, and their inventors do not have necessarily studied how to eliminate or inhibit the aforementioned risks of the onset of adverse events (the first problem described above) and reduced bioavailability due to the sustained release characteristics (the second problem described above). That is, there is no disclosure of a preparation which not only inhibits the dissolution of the basic drug or the salt thereof in accordance with the environment in the body so that the initial drug burst (rapid release of the drug immediately after dissolution) is suppressed, but also reliably provides dissolution with low pH-dependence at the early stage of the dissolution test, and wherein a ratio of the dissolution rate of the basic drug or the salt thereof in an acidic test solution to the dissolution rate of the basic drug or the salt thereof in a neutral test solution (dissolution rate in the acidic test solution / dissolution rate in the neutral test solution) decreases with dissolution time at the late stage of dissolution, as compared to the early stage of dissolution.

### Disclosure of Invention

### Problems to be Solved by the Invention

Taking a pH environment in the body into consideration, there is a demand for the sustained-release preparations containing a basic drug, which inhibit unexpected increases in blood concentrations associated with the rapid dissolution of the basic drug from the preparations and which offer a decreased risk of reduced bioavailability associated with the sustained-release characteristics. That is, there is a demand for a matrix type sustained-release preparation containing a basic drug or a salt thereof, which not only inhibits the initial drug burst (rapid drug release immediately after dissolution) in a dissolution test, but also ensures dissolution with low pH dependence of the basic drug or the salt thereof at the early stage of dissolution, and wherein as the dissolution test proceeds, a dissolution speed in a neutral test solution is high at the late stage of dissolution. Accordingly, this is a demand for the matrix type sustained-release preparation containing a basic drug or a salt thereof in which a ratio of a dissolution rate of the basic drug or the salt thereof in an acidic test solution to a dissolution rate of the basic drug or the salt thereof in a neutral test solution (dissolution rate in the acidic test solution / dissolution rate in the neutral test solution) decreases with dissolution time at the late stage of dissolution, as compared to the early stage of dissolution. In particular, there is a demand for a matrix type sustained-release preparation which is capable of controlling the dissolution of the basic drug or the salt thereof, so that the solubility of the basic drug or the salt thereof decreases greatly with increased pH from a near-neutral to a weakly alkaline.

### Means for Solving the Problems

In view of these circumstances, the present inventors have discovered as a result of exhaustive research that desired objects can be achieved with the construction shown below, so as to reach at the present invention:

In other words, in one aspect of the present invention, there is provide (I) a matrix type sustained-release preparation comprising (1) a basic drug or a salt thereof which has higher solubility in a 0.1 N hydrochloric acid solution and a neutral aqueous solution, pH 6.0 than in a basic aqueous solution, pH 8.0; and (2) at least one enteric polymer. In this aspect, the neutral aqueous solution is 50 mM phosphate buffer, and the basic aqueous solution is 50 mM phosphate buffer.

The present invention also provides (II) the matrix type sustained-release preparation according to item (I), wherein in a dissolution test according to the Japanese Pharmacopoeia paddle method for dissolution tests, a ratio of a dissolution rate of the basic drug or the salt thereof in the 0.1 N hydrochloric acid solution to a dissolution rate of the basic drug or the salt thereof in the 50 mM phosphate buffer, pH 6.8 decreases with dissolution time until a dissolution time at which the dissolution rate of the basic drug or the salt thereof in the 50 mM phosphate buffer, pH 6.8 is 90%.

According to a preferred aspect of the present invention, there is provided (III) the matrix type sustained-release preparation according to item (I) or (II), wherein in the dissolution test according to the Japanese Pharmacopoeia paddle method for the dissolution tests, the dissolution rate of the basic drug or the salt thereof in the 0.1 N hydrochloric acid solution is not more than 60% at a dissolution time of 1 hour. The dissolution rate of the basic drug or the salt thereof in the 0.1 N hydrochloric acid solution is more preferably not more than 50% at a dissolution time of 1 hour, still more preferably not more than 40%.

According to the more preferred aspect of the present invention, there is provided (IV) the matrix type sustained-release preparation according to any one of items (I) to (III), wherein in the dissolution test according to the Japanese Pharmacopoeia paddle method for the dissolution test, the ratio of the dissolution rate of the basic drug or the salt thereof in the 0.1 N hydrochloric acid solution to the dissolution rate of the basic drug or the salt thereof in the 50 mM phosphate buffer, pH 6.8 is from 0.3 to 1.5 at a dissolution time of 3 hours. More preferably, the ratio of the dissolution rate is from 0.3 to 1.4, still more preferably from 0.3 to 1.3, most preferably from 0.3 to 1.2.

According to the still more preferred aspect of the present invention, there is provided (V) the matrix type sustained-release preparation according to any one of items (I) to (IV), wherein in the dissolution test according to the Japanese Pharmacopoeia paddle method for the dissolution test, the dissolution rate of the basic drug or the salt thereof in the 0.1 N hydrochloric acid solution is not more than 60% at a dissolution time of 1 hour, and the ratio of the dissolution rate of the basic drug or the salt thereof in the 0.1 N hydrochloric acid solution to the dissolution rate of the basic drug or the salt thereof in the 50 mM phosphate buffer, pH 6.8 is from 0.3 to 1.5 at a dissolution time of 3 hours. More preferably, the dissolution rate of the basic drug or the salt thereof in the 0.1. N hydrochloric acid solution is not more than 50% at a dissolution time of 1 hour and the ratio of the dissolution rates is from 0.3 to 1.4, still more preferably the dissolution rate of the basic drug or the salt thereof in the 0.1 N hydrochloric acid solution being not more than 40% and the ratio of the dissolution rates being from 0.3 to 1.2.

The matrix type sustained-release preparation according to the present invention may also comprise at least one water-insoluble polymer. For example, the present invention provides a matrix type sustained-release preparation comprising: (1) a basic drug or a salt thereof which has higher solubility in a 0.1 N hydrochloric acid solution and a 50 mM phosphate buffer, pH 6.0 than in a 50 mM phosphate buffer, pH 8.0; (2) at least one enteric polymer; and (3) at least one water-insoluble polymer.

According to a more preferred aspect of the present invention, there is provided the matrix type sustained-release preparation comprising (1) the basic drug or the salt thereof in which solubility of the basic drug or the salt thereof in the neutral aqueous solution, pH 6.8 is at least twice its solubility in the basic aqueous solution, pH 8.0 and is not more than half its solubility in the neutral aqueous solution, pH 6.0; and (2) at least one enteric polymer. Alternatively, the matrix type sustained-release preparation also comprises (3) at least one water-insoluble polymer. According to a particularly preferred aspect of the present invention, there is provided the matrix type sustained-release preparation comprising (1) the basic drug or the salt thereof in which solubility of the basic drug or the salt thereof in the 50 mM phosphate buffer, pH 6.8 is at least twice its solubility in the 50 mM phosphate buffer, pH 8.0 and is not more than half its solubility in the 50 mM phosphate buffer, pH 6.0; and (2) at least one enteric polymer. Alternatively, the matrix type sustained-release preparation also comprises (3) at least one water-insoluble polymer.

According to a particularly preferred aspect of the present invention, there is also provided the matrix type sustained-release preparation comprising (1) the basic drug or the salt thereof in which solubility of the basic drug or the salt thereof in the 0.1 N hydrochloric acid solution and the 50 mM phosphate buffer, pH 6.0 is 1 mg/mL or more and the solubility of the basic drug or the salt thereof in the 50 mM phosphate buffer, pH 8.0 is 0.2 mg/mL or less; (2) at least one enteric polymer. Alternatively, the matrix type sustained-release preparation also comprises (3) at least one water-insoluble polymer.

According to a particularly preferred aspect of the present invention, there is also provided the matrix type sustained-release preparation comprising (1) the basic drug or the salt thereof in which the solubility of the basic drug or the salt thereof in the 0.1 N hydrochloric acid solution and the 50 mM phosphate buffer, pH 6.0 is 1 mg/mL or more, the solubility of the basic drug or the salt thereof in the 50 mM phosphate buffer, pH 8.0 is 0.2 mg/mL or less, and the solubility of the basic drug or the salt thereof in the 50 mM phosphate buffer, pH 6.8 is at least twice its solubility in the 50 mM phosphate buffer, pH 8.0 and is not more than half its solubility in the 50 mM phosphate buffer, pH 6.0; (2) at least one enteric polymer. Alternatively, the matrix type sustained-release preparation also comprises (3) at least one water-insoluble polymer.

According to a particularly preferred aspect of the present invention, there is also provided the matrix type sustained-release preparation comprising: (1) the basic drug or the salt thereof in which solubility of the basic drug or the salt thereof is 1 mg/mL or more in the 0.1 N hydrochloric acid solution and the 50 mM phosphate buffer, pH 6.0 and is 0.2 mg/mL or less in the 50 mM phosphate buffer, pH 8.0, and the solubility of the basic drug or the salt thereof in the 50 mM phosphate buffer, pH 6.8 is at least twice its solubility in the 50 mM phosphate buffer, pH 8.0 and is not more than half its solubility in the 50 mM phosphate buffer, pH 6.0; and (2) at least one enteric polymer. Alternatively, the matrix type sustained-release preparation also comprises (3) at least one water-insoluble polymer.

### Advantageous Effects of the Invention

According to the present invention, in a matrix type sustained-release preparation containing a basic drug or a salt thereof, which has higher solubility in the 0.1 N hydrochloric acid solution and the neutral aqueous solution, pH 6.0 than in the basic aqueous solution, pH 8.0, the pH dependence of dissolution of the drug or the salt thereof at the early stage of dissolution is reduced, and the ratio of the dissolution rate of the drug or the salt thereof in the acidic test solution to the dissolution rate of the drug or the salt thereof in the neutral test solution (dissolution rate in the acidic test solution / dissolution rate in the neutral test solution) decreases with dissolution time as the dissolution test proceeds (the ratio being lower at the late stage than at the early stage of the dissolution test). From these dissolution behaviors, the risk of adverse events due to the sustained release characteristics at the early stage of dissolution can be reduced, and the risk of reduced bioavailability can also be inhibited. Furthermore, since 90% or more of the drug contained in the preparation according to the present invention can be released in the neutral test solution within 8 hours which is the estimated as the upper limit of large intestinal arrival time in humans, there is little risk of decreased bioavailability due to the sustained release characteristics, and it is believed that the preparations according to the present invention are extremely useful. Advantageous effects of the present invention are shown below.

### Brief Description of Drawings

Figure 1 shows the results of dissolution profiles of donepezil hydrochloride in 0.1 N hydrochloric acid solutions in the matrix type sustained-release preparations of Examples 2 and 4 according to the present invention (control experiment: Comparative Example 1);
Figure 2 shows the results of dissolution profiles of donepezil hydrochloride in the 50 mM phosphate buffer, pH 6.8 in the matrix sustained-release preparations of Examples 2 and 4 according to the present invention (control experiment: Comparative Example 1);
Figure 3 shows the results of dissolution profiles of donepezil hydrochloride in 0.1 N hydrochloric acid solutions in the matrix type sustained-release preparations of Examples 14 through 17 according to the present invention;
Figure 4 shows the results of dissolution profiles of donepezil hydrochloride in the 50 mM phosphate buffer, pH 6.8 in the matrix type sustained-release preparations of Examples 14 through 17 according to the present invention;
Figure 5 shows the results of dissolution profiles of donepezil hydrochloride in 0.1 N hydrochloric acid solutions in the matrix type sustained-release preparations of Examples 12 and 13 according to the present invention (control experiment: Comparative Example 2);
Figure 6 shows the results of dissolution profiles of donepezil hydrochloride in the 50 mM phosphate buffer, pH 6.8 in the matrix type sustained-release preparations of Examples 12 and 13 according to the present invention (control experiment: Comparative Example 2).;
Figure 7 shows the results of dissolution profiles of donepezil hydrochloride in test solution A and test solution B in the matrix type sustained-release preparations of Example 27 according to the present invention;
Figure 8 shows the results of dissolution profiles of donepezil hydrochloride in test solution A and test solution B in the matrix type sustained-release preparations of Examples 28 and 29 according to the present invention; and
Figure 9 shows the results of dissolution profiles of donepezil hydrochloride in test solution A and test solution B in the matrix type sustained-release preparations of Examples 30 and 31 according to the present invention.

### Best Mode for Carrying Out the invention

The present invention will be explained in detail below by way of examples and comparative examples, but the present invention is not limited thereto.

There are no particular limitations on the basic drug or the salt thereof used in the present invention. For example, the salt of the basic drug used in the present invention may be either an organic or inorganic acid salt. Examples of the salt include, but are not limited to, hydrochlorides, sulfates, acetates, phosphates, carbonates, mesylates, tartrates, citrates, tosylates and the like. Examples of the basic drug or the salt thereof used in the present invention include, but are not limited to, anti-dementia drugs such as donepezil hydrochloride, galantamine hydrobromide, rivastigmine tartrate, memantine hydrochloride, tacrine and the like; anti-anxiety drugs such as flurazepam hydrochloride, alprazolam, tandospirone citrate, rilmazafone hydrochloride and the like; antihistamines such as diphenylpyraline hydrochloride, chlorpheniramine maleate, cimetidine, isothipendyl hydrochloride and the like; circulatory drugs such as phenylephrine hydrochloride, procainamide hydrochloride, quinidine sulfate, isosorbide dinitrate, nicorandil and the like; antihypertensive drugs such as amlodipine besylate, nifedipine, nicardipine hydrochloride, nilvadipine, atenolol hydrochloride and the like; anti-psychotic drugs such as perospirone hydrochloride and the like; anti-bacterial agents such as levofloxacin and the like; antibiotics such as cephalexin, cefcapene pivoxil hydrochloride, ampicillin and the like as well as sulfamethoxazole, tetracycline, metronidazole, indapamide, diazepam, papaverine hydrochloride, bromhexine hydrochloride, ticlopidine hydrochloride, carbetapentane citrate, phenylpropanolamine hydrochloride, ceterizine hydrochloride and other drugs and macrolide antibiotics such as erythromycin, dirithromycin, josamycin, midecamycin, kitasamycin, roxithromycin, rokitamycin, oleandomycin, miokamycin, flurithromycin, rosaramycin, azithromycin, clarithromycin and the like. Either one or two or more of these basic drugs or salts thereof may be contained in the matrix type sustained-release preparation according to the present invention.

Of these basic drugs or salts thereof, the anti-dementia drugs are preferred, and donepezil hydrochloride and / or memantine hydrochloride are particularly preferred. The matrix type sustained-release preparation according to the present invention may be also suitable for the basic drugs or the salts thereof which have a narrow drug safety range or which produce adverse effects dependent on maximum blood concentration of the drug. There are no particular limitations on the anti-dementia drug contained in the matrix type sustained-release preparation according to the present invention, but from the standpoint of controlling release it is effective for the basic drugs or the salts thereof which are less soluble in an alkaline aqueous solution than in an acidic aqueous solutions and the solubility of the basic drugs or the salts thereof for a pH of an aqueous solution changes near the neutral pH. Examples include the basic drugs or the salts thereof with a pKa of 7.0 to 12, preferably 7.5 to 11, more preferably 8.0 to 10.5, still more preferably 8.5 to 10.5. For example, donepezil hydrochloride is a basic drug with a pKa of 8.90 and memantine hydrochloride is a basic drug with a pKa of 10.27.

There are no particular limitations on the solubility of the basic drug or the salt thereof used in the present invention with respect to acidic aqueous solutions, neutral aqueous solutions or basic aqueous solutions, but the solubility of the basic drug or the salt thereof in the acidic aqueous solution and the neutral aqueous solution is higher than its solubility in the basic aqueous solution. Herein, for use in preparation of these aqueous solutions, examples for this use includes, but are not limited to, a phosphate buffer (for instance, buffers prepared with 50 mM sodium phosphate solution and hydrochloric acid), buffers such as G. L. Miller's buffer, Atkins-Pantin's buffer, Good's buffer or the like, 0.1 N hydrochloric acid, 0.1 mol/L sodium hydroxide solution or the like. Note that the solubility used in the present invention refers to solubility wherein a solution temperature is 25 °C.

The term "solubility in an acidic aqueous solution" used in the present invention means that a solubility of the basic drug or the salt thereof in a solution exhibiting an acidic property when dissolving the basic drug or the salt thereof in a buffer or the like. Similarly, the term "solubility in a neutral (basic) aqueous solution used in the present invention means that a solubility of the basic drug or the salt thereof in a solution exhibiting a neutral (basic) property when dissolving the basic drug or the salt thereof in a buffer or the like.

By way of example, the basic drug or the salt thereof used in the present invention has a higher solubility in the acidic aqueous solution, pH 3.0 and the neutral aqueous solution, pH 6.0 than in the basic aqueous solution, pH 8.0. The term "solubility in the acidic aqueous solution, pH 3.0" used herein means that a solubility of the basic drug or the salt thereof in the acidic solution having a pH 3.0 when dissolving the basic drug or the salt thereof in a buffer or the like. The term "solubility in the neutral aqueous solution, pH 6.0" used herein means that a solubility of the basic drug or the salt thereof in a solution having a pH 6.0 when dissolving the basic drug or the salt thereof in a buffer or the like. Similarly, the term "solubility in the basic aqueous solution, pH 8.0" used herein means that a solubility of the basic drug or the salt thereof in a solution having a pH 8.0 when dissolving the basic drug or the salt thereof in a buffer or the like.

By way of another example, the basic drug or the salt thereof used in the present invention has a higher solubility in a 0.1 N hydrochloric acid solution and the neutral aqueous solution, pH 6.0 than in the basic aqueous solution, pH 8.0. The term "solubility in the 0.1 N hydrochloric acid solution" used herein means a solubility of the basic drug or the salt thereof when dissolving the basic drug or the salt thereof in the 0.1 N hydrochloric acid solution. For example, the solutions of donepezil hydrochloride and memantine hydrochloride which are dissolved in the 0.1 N hydrochloric acid solution show a pH range of from about 1 to about 2.

Preferably, the basic drug or the salt thereof used in the present invention has a solubility in the 0.1 N hydrochloric acid solution and the neutral aqueous solution, pH 6.0 being higher than in the basic aqueous solution, pH 8.0 and a solubility in the neutral aqueous solution, pH 6.8 being at least twice its solubility in the basic aqueous solution, pH 8.0, and being not more than half its solubility in the neutral aqueous solution, pH 6.0. The term "solubility in the neutral aqueous solution, pH 6.8" used herein means that a solubility of the basic drug or the salt thereof in a solution having a pH 6.8 when dissolving the basic drug or the salt thereof in a buffer or the like.

More specifically, there are no particular limitations as long as the solubility of the basic drug or the salt thereof in the 0.1 N hydrochloric acid solution and the neutral aqueous solution, pH 6.0 is 1 mg/mL or more, the solubility of the basic drug or the salt thereof in the basic aqueous solution, pH 8.0 is 0.2 mg/mL or less, and the solubility of the basic drug or the salt thereof in the neutral aqueous solution, pH 6.8 is two or more times its solubility in the basic aqueous solution, pH 8.0 and is not more than half its solubility in the neutral aqueous solution, pH 6.0. That is, the solubility of the basic drug or the salt thereof in the 0.1 N hydrochloric acid solution and the neutral aqueous solution, pH 6.0 is not particularly limited as long as the above solubility is 1 mg/mL or more. The above solubility is generally 1 to 1000 mg/mL, preferably 5 to 200 mg/mL, more preferably 5 to 100 mg/mL, still more preferably 10 to 80 mg/mL. The solubility of the basic drug or the salt thereof in the basic aqueous solution, pH 8.0 is not particularly limited as long as the above solubility is 0.2 mg/mL or less. The above solubility is generally 0.0001 to 0.2 mg/mL, preferably 0.0005 to 0.1 mg/mL, more preferably 0.001 to 0.05 mg/mL, still more preferably 0.002 to 0.03 mg/mL. Moreover, the solubility of the basic drug or the salt thereof in the neutral aqueous solution, pH 6.8 is not particularly limited as long as the above solubility is at least twice its solubility in the basic aqueous solution, pH 8.0 and is not more than 1/2 solubility in the neutral aqueous solution, pH 6.0. The above solubility is preferably at least 3 times solubility in the basic aqueous solution, pH 8.0 and is not more than 1/3 solubility in the neutral aqueous solution, pH 6.0, more preferably at least 5 times solubility in the basic aqueous solution, pH 8.0 and not more than 1/5 solubility in the neutral aqueous solution, pH 6.0, still more preferably at least 10 times solubility in the basic aqueous solution, pH 8.0 and not more than 1/10 solubility in the neutral aqueous solution, pH 6.0.

By way of still another example, the solubility of the basic drug or the salt thereof used in the present invention in the 0.1 N hydrochloric acid solution and the 50 mM phosphate buffer, pH 6.0 is higher than its solubility in the 50 mM phosphate buffer, pH 8.0. The term "solubility in the 50 mM phosphate buffer, pH 6.0" used herein means a solubility of the basic drug or the salt thereof in the 50 mM phosphate buffer having pH 6.0 when dissolving the basic drug or the salt thereof in the 50 mM phosphate buffer. Similarly, the term "solubility in the 50 mM phosphate buffer, pH 8.0" used herein means a solubility of the basic drug or the salt thereof in the 50 mM phosphate buffer having pH 8.0 when dissolving the basic drug or the salt thereof in the 50 mM phosphate buffer.

Preferably, the solubility of the basic drug or the salt thereof in the 0.1 N hydrochloric acid solution and the 50 mM phosphate buffer, pH 6.0 is higher than its solubility in the 50 mM phosphate buffer, pH 8.0, and the solubility in the 50 mM phosphate buffer, pH 6.8 is two or more times its solubility in the 50 mM phosphate buffer, pH 8.0 and is not more than half its solubility in the 50 mM phosphate buffer, pH 6.0. To be more specific, there are no particular limitations as long as the solubility of the basic drug or the salt thereof in the 0.1 N hydrochloric acid solution and the 50 mM phosphate buffer, pH 6.0 is 1 mg/mL or more, the solubility of the basic drug or the salt thereof in the 50 mM phosphate buffer, pH 8.0 is 0.2 mg/mL or less, and the solubility of the basic drug or the salt thereof in the 50 mM phosphate buffer, pH 6.8 is two or more times its solubility in the 50 mM phosphate buffer, pH 8.0 and is not more than half its solubility in the 50 mM phosphate buffer, pH 6.0. That is, the solubility of the basic drug or the salt thereof in the 0.1 N hydrochloric acid solution and the 50 mM phosphate buffer, pH 6.0 is not particularly limited as long as the above solubility is 1 mg/mL or more. The above solubility is generally 1 to 1000 mg/mL, preferably 5 to 200 mg/mL, more preferably 5 to 100 mg/mL, still more preferably 10 to 80 mg/mL. The solubility of the basic drug or the salt thereof in the 50 mM phosphate buffer, pH 8.0 is not particularly limited as long as the above solubility is 0.2 mg/mL or less. The above solubility is generally 0.0001 to 0.2 mg/mL, preferably 0.0005 to 0.1 mg/mL, more preferably 0.001 to 0.05 mg/mL, still more preferably 0.002 to 0.03 mg/mL. Moreover, the solubility of the basic drug or the salt thereof in the 50 mM phosphate buffer, pH 6.8 is not particularly limited as long as the above solubility is at least twice its solubility in the 50 mM phosphate buffer, pH 8.0 and is not more than 1/2 solubility in the 50 mM phosphate buffer, pH 6.0. The above solubility is preferably at least 3 times solubility in the 50 mM phosphate buffer, pH 8.0 and is not more than 1/3 solubility in the 50 mM phosphate buffer, pH 6.0, more preferably at least 5 times solubility in the 50 mM phosphate buffer, pH 8.0 and not more than 1/5 solubility in the 50 mM phosphate buffer, pH 6.0, still more preferably at least 10 times solubility in the 50 mM phosphate buffer, pH 8.0 and not more than 1/10 solubility in the 50 mM phosphate buffer, pH 6.0.

For example, donepezil hydrochloride is characterized by its solubility of 11 to 16 mg/mL in an acidic aqueous solution, pH 3.0 and a neutral aqueous solution, pH 6.0 and 0.1 mg/mL or less in a basic aqueous solution, pH 8.0. Moreover, donepezil hydrochloride is a weakly basic drug or a salt thereof having one tertiary amino group, which has been widely used for Alzheimer's disease dementia, and is characterized by its solubility in the neutral aqueous solution, pH 6.8 being at least twice solubility in the basic aqueous solution, pH 8.0 and being not more than 1/2 solubility in the neutral aqueous solution, pH 6.0.

Alternatively, donepezil hydrochloride is a weakly basic drug or a salt thereof having one tertiary amino group, which has been widely used for Alzheimer's disease dementia. Donepezil hydrochloride is characterized by its solubility of 11 to 16 mg/mL in the 0.1 N hydrochloric acid solution and the 50 mM phosphate buffer, pH 6.0 and 0.1 mg/mL or less in the 50 mM phosphate buffer, pH 8.0, with the solubility in the 50 mM phosphate buffer, pH 6.8 being at least twice solubility in the 50 mM phosphate buffer, pH 8.0 and being not more than 1/2 solubility in the 50 mM phosphate buffer, pH 6.0.

There are no particular limitations on the dose of the weak basic drug or the salt thereof used in the present invention, depending on the species of the weak basic drug and aspect of the patient of each disorder, but in the case of the acetylcholinesterase inhibitor for Alzheimer-type dementia, the dose is from 0.01 to 50 mg/day. More specifically, the dose of donepezil or a pharmacologically acceptable salt thereof is from 0.01 to 50 mg/day, preferably from 0.1 to 40 mg/day, more preferably from 1 to 30 mg/day, still more preferably from 5 to 25 mg/day. The dose of rivastigmine or a pharmacologically acceptable salt thereof is from 0.01 to 50 mg/day, preferably from 0.1 to 30 mg/day, more preferably from 1 to 20 mg/day, still more preferably from 1 to 15 mg/day. The dose of galantamine or a pharmacologically acceptable salt thereof is from 0.01 to 50 mg/day, preferably from 0.1 to 40 mg/day, more preferably from 1 to 30 mg/day, still more preferably from 2 to 25 mg/day.

Moreover, in the case of memantine or a pharmacologically acceptable salt thereof which acts as a N-methyl-D-asparate (NMDA) receptor antagonist, the dose is from 0.5 to 100 mg/day, preferably from 1 to 100 mg/day, more preferably from 1 to 40 mg/day, still more preferably from 5 to 25 mg/day.

There are no particular limitations on the enteric polymer used in the present invention, but preferably it should dissolve in some aqueous buffer solutions at a pH somewhere in the range of 5.0 to 8.0, although the enteric polymer does not dissolve in the 0.1 N hydrochloric acid solution. At least one enteric polymer can be used, and two or more enteric polymers may be mixed. Examples of the enteric polymers include, but are not limited to, methacrylic acid-methyl methacrylate copolymer (Eudragit L100, Eudragit S100 etc.: Röhm GmbH & Co. KG, Darmstadt, Germany), methacrylic acid-ethyl acrylate copolymer (Eudragit L100-55, Eudragit L30D-55 etc.: Röhm GmbH & Co. KG, Darmstadt, Germany), hydroxypropyl methylcellulose phthalate (HP-55, HP-50 etc.: Shin-Etsu Chemical, Japan), hydroxypropyl methylcellulose acetate succinate (AQOAT etc.: Shin-Etsu Chemical, Japan), carboxymethyl ethylcellulose (CMEC: Freund Corporation, Japan), cellulose acetate phthalate and the like, with methacrylic acid-ethyl acrylate copolymer, methacrylic acid-methyl methacrylate copolymer, hydroxypropyl methylcellulose phthalate and hydroxypropyl methylcellulose acetate succinate being preferred. The enteric polymer which can dissolve in a buffer solution at a pH in the range of from 5.0 to 6.8 is more preferable. Examples of such enteric polymers include, but are not limited to, methacrylic acid-ethyl acrylate copolymer and hydroxypropyl methylcellulose acetate succinate. In particular, it is particularly preferable that methacrylic acid-ethyl acrylate be Eudragit L100-55 (Röhm GmbH & Co. KG, Darmstadt, Germany), which is a powder and which can dissolve in a buffer solution at a pH in a range of not less than 5.5. Moreover, it is particularly preferable that hydroxypropyl methylcellulose acetate succinate be AQOAT LF (which can dissolve at a pH in a range of not less than 5.5; Shin-Etsu Chemical, Japan) or AQOAT MF (which can dissolve at a pH in a range of not less than 6.0; Shin-Etsu Chemical, Japan), which are fine powders and which have a mean particle size being about 5 µm. Note that there are no particular limitations on the mean particle size of the enteric polymer used in the present invention, but generally the smaller the better, and the mean particle size is preferably 0.05 to 100 µm, more preferably 0.05 to 70 µm, still more preferably 0.05 to 50 µm.

The water-insoluble polymer used in the present invention refers to a sustained-release base which does not dissolve in an aqueous buffer solution at a pH anywhere in the range of 1.0 to 8.0, and is not particularly limited. The matrix type sustained-release preparation according to the present invention preferably comprises at least one water-insoluble polymer, and two or more water-insoluble polymers may be contained in the matrix type sustained-release preparation.

Examples of the water-insoluble polymers include, but are not limited to, cellulose ethers (cellulose alkyl ethers such as ethylcellulose, ethyl methylcellulose, ethyl propylcellulose, isopropylcellulose, butylcellulose and the like; cellulose aralkyl ethers such as benzyl cellulose and the like; cellulose cyanoalkyl ethers such as cyanoethyl cellulose and the like), cellulose esters (cellulose organic acid esters such as cellulose acetate butyrate, cellulose acetate, cellulose propionate, cellulose butyrate, cellulose acetate propionate and the like), methacrylic acid-acrylic acid copolymers (trade names: Eudragit RS, Eudragit RL, Eudragit NE, Röhm GmbH & Co. KG, Darmstadt, Germany) and the like. Of these polymers, ethylcellulose, aminoalkyl methacrylate copolymer RS (Eudragit RL, Eudragit RS) and ethyl acrylate-methyl methacrylate copolymer (Eudragit NE) are preferred and ethylcellulose (ETHOCEL, The Dow Chemical Company, U.S. and the like) is most preferred. There are no particular limitations on the mean particle size of the water-insoluble polymer used in the present invention, but generally the smaller the better. The mean particle size is preferably 0.1 to 100 µm, more preferably 1 to 50 µm, still more preferably 3 to 15 µm, most preferably 5 to 15 µm.

An amount of the enteric polymer in the matrix type sustained-release preparation is not particularly limited, but is generally 5 to 90% by weight, preferably 8 to 70% by weight, more preferably 10 to 60% by weight, most preferably 15 to 50% by weight, based on 100% by weight of the matrix type sustained-release preparation. A total amount of the water-insoluble polymer and the enteric polymer in the matrix type sustained-release preparation is not particularly limited, but is generally 25 to 95% by weight, preferably 35 to 95% by weight, more preferably 35 to 90% by weight, most preferably 35 to 75% by weight, based on 100% by weight of the matrix type sustained-release preparation.

In the matrix type sustained-release preparation according to the present invention it is preferable that the water-insoluble polymer be ethylcellulose and that the enteric polymer be at least one selected from the group consisting of methacrylic acid-ethyl acrylate copolymer, methacrylic acid-methyl methacrylate copolymer and hydroxypropyl methylcellulose acetate succinate. It is more preferable that the water-insoluble polymer be ethylcellulose and that the enteric polymer be methacrylic acid-ethyl acrylate copolymer and / or hydroxypropyl methylcellulose acetate succinate.

An amount of the water-insoluble polymer in the matrix type sustained-release preparation is not particularly limited, but is generally 1 to 90% by weight, preferably 3 to 70% by weight, more preferably 5 to 50% by weight, still more preferably 5 to 35% by weight, based on 100% by weight of the matrix type sustained-release preparation.

The matrix type sustained-release preparation according to the present invention provides remarkable features that dissolution with low pH dependence of the basic drug or the salt thereof at the early stage of dissolution can be ensured in the dissolution test and that as the dissolution test proceeds, a ratio of a dissolution rate of the basic drug or the salt thereof in an acidic dissolution test solution (hereinafter referred to as an acidic test solution) to a dissolution rate of the basic drug or the salt thereof in a neutral dissolution test solution (hereinafter referred to as a neutral test solution) (dissolution rate in the acidic test solution / dissolution rate in the neutral test solution) decreases with dissolution time at the late stage of dissolution, as compared to the early stage of dissolution. In the matrix type sustained-release preparation according to the present invention, by mixing the enteric polymer with the basic drug or the salt thereof having higher solubility in the acidic aqueous solution and the neutral aqueous solution than in the basic aqueous solution described above, dissolution of the basic drug or the salt thereof can be inhibited in the acidic and neutral dissolution test solutions. When mixing with the water-insoluble polymer and the enteric polymer, the greater the amount of enteric polymer mixed with the water-insoluble polymer the greater the reduction in dissolution speed of the basic drug or the salt thereof in the acidic and neutral dissolution test solutions, thus easily providing a matrix type sustained-release preparation wherein dissolution with low pH dependence of the basic drug or the salt thereof at the early stage of dissolution can be ensured in the dissolution test, and wherein as the dissolution test proceeds, the ratio of the dissolution rate of the basic drug or the salt thereof in the acidic test solution to the dissolution rate of the basic drug or the salt thereof in the neutral test solution (dissolution rate in the acidic test solution / dissolution rate in the neutral test solution) decrease with dissolution time (specifically the ratio decreases at the late stage of dissolution, as compared to the early stage of dissolution).

Herein, characteristic features of the matrix type sustained-release preparation according to the present invention can be specifically demonstrated by dissolution profile in a 50 mM phosphate buffer, pH 6.8 as the neutral dissolution test solution and in 0.1 N hydrochloric acid solution as the acidic dissolution test in the dissolution test. That is, more specifically, when the basic drug or the salt thereof is released from the matrix type sustained-release preparation according to the present invention in a dissolution test according to the Japanese Pharmacopoeia paddle method for dissolution tests, the ratio of the dissolution rate of the basic drug or the salt thereof in the 0.1 N hydrochloric acid solution to the dissolution rate of the basic drug or the salt thereof in the 50 mM phosphate buffer, pH 6.8 decreases with dissolution time until a dissolution time at which the dissolution rate of the basic drug or the salt thereof in the 50 mM phosphate buffer, pH 6.8 is 90%. Moreover, the present invention provides the matrix type sustained-release preparation wherein in the dissolution test according to the Japanese Pharmacopoeia paddle method for the dissolution tests, the dissolution rate of the basic drug or the salt thereof in the 0.1 N hydrochloric acid solution at a dissolution time of 1 hour is not more than 60%, preferably not more than 50%, more preferably not more than 40%. In addition, at the early stage of dissolution in the dissolution test according to the Japanese Pharmacopoeia paddle method for the dissolution tests, the ratio of the dissolution rate of the basic drug or the salt thereof in the 0.1 N hydrochloric acid solution to the dissolution rate of the basic drug or the salt thereof in the 50 mM phosphate buffer, pH 6.8 is from 0.3 to 1.5, preferably from 0.3 to 1.4, more preferably from 0.3 to 1.3, still more preferably from 0.3 to 1.2 at a dissolution time of 3 hours. Note that the Japanese Pharmacopoeia paddle method for dissolution tests is described in the Japanese Pharmacopoeia, 14^{th} edition, and for example, the test can be performed at a paddle rate of 50 rpm.

The matrix type sustained-release preparation according to the present invention preferably also comprises a water-soluble sugar and / or a water-soluble sugar alcohol. There are no particular limitations on the water-soluble sugar and / or the water-soluble sugar alcohol. Examples of the water-soluble sugars include, but are not limited to, lactose, sucrose, glucose, dextrin, pullulan and the like. Examples of the water-soluble sugar alcohols include, but are not limited to, mannitol, erythritol, xylitol, sorbitol and the like, with lactose and mannitol being preferred. There are no particular limitations on the amount of the water-soluble sugar or water-soluble sugar alcohol in the matrix type sustained-release preparation according to the present invention, but the above amount is generally 3 to 70% by weight, preferably 5 to 60% by weight, more preferably 10 to 60% by weight, still more preferably 12 to 60% by weight, based on 100% by weight of the matrix type sustained-release preparation.

The matrix type sustained-release preparation according to the present invention further comprises a variety of pharmacologically acceptable carriers, such as diluents, lubricants, binders, disintegrators and the like as well as other preparation additives such as preservatives, anti-oxidants, colorants, sweeteners, plasticizers and the like, if necessary. The prepared matrix type sustained-release preparation can also be given a film coating as necessary. Examples of diluents include, but are not limited to, starch, pregelatinized starch, crystalline cellulose, light anhydrous silicic acid, synthetic aluminum silicate, magnesium aluminate metasilicate and the like. Examples of lubricants include, but are not limited to, magnesium stearate (Mallinckrodt Baker, Inc. USA), calcium stearate, talc, sodium stearyl fumarate and the like. Examples of binders include hydroxypropylcellulose, methylcellulose, carboxymethylcellulose sodium, hydroxypropyl methylcellulose, polyvinylpyrrolidone and the like. Examples of disintegrators include, but are not limited to, carboxymethyl cellulose, carboxymethyl cellulose calcium, croscarmellose sodium, carboxymethyl starch sodium, low-substituted hydroxypropylcellulose and the like. Examples of preservatives include, but are not limited to, paraoxybenzoic acid esters, chlorobutanol, benzyl alcohol, phenethyl alcohol, dehydroacetic acid, sorbic acid and the like. Examples of anti-oxidants include, but are not limited to, sulfites, ascorbates and the like. Favorable examples of colorants include, but are not limited to, non-water-soluble lake pigments, natural pigments (such as β-carotene, chlorophyll and red ferric oxide), yellow ferric oxide, red ferric oxide, black iron oxide and the like. Examples of sweeteners include, but are not limited to, sodium saccharin, dipotassium glycyrrhizate, aspartame, stevia and the like. Examples of plasticizers include, but are not limited to, glycerin fatty acid esters (trade name Myvacet), triethyl citrate (trade name Citroflex 2), propylene glycol, polyethylene glycol and the like. Examples of film coating bases include, but are not limited to, hydroxypropyl methylcellulose, hydroxypropyl cellulose and the like.

An amount of the carrier contained in the matrix type sustained-release preparation according to the present invention is not particularly limited, but for example, an amount of the lubricant is generally from 0 to 5% by weight, preferably from 0.01 to 4% by weight, more preferably from 0.1 to 3% by weight, still more preferably from 0.3 to 1 % by weight, based on 100 % by weight of the matrix type sustained-release preparation. Moreover, an amount of the binder is generally from 0 to 10% by weight, preferably from 0.1 to 8% by weight, more preferably from 0.5 to 6% by weight, still more preferably from 1 to 3% by weight, based on 100 % by weight of the matrix type sustained-release preparation.

The matrix type sustained-release preparation according to the present invention comprises, for example, (a) a basic drug or a salt thereof, (b) 5 to 90% by weight of an enteric polymer and (c) 3 to 70% by weight of a water-soluble sugar alcohol, based on 100% by weight of the matrix type sustained-release preparation. Preferably, the matrix type sustained-release preparation according to the present invention comprises (a) a basic drug or a salt thereof, (b) 8 to 70% by weight of an enteric polymer and (c) 5 to 60% by weight of a water-soluble sugar alcohol, based on 100% by weight of the matrix type sustained-release preparation. More preferably, the matrix type sustained-release preparation according to the present invention comprises (a) a basic drug or a salt thereof, (b) 10 to 60% by weight of an enteric polymer and (c) 10 to 60% by weight of a water-soluble sugar alcohol, based on 100% by weight of the matrix type sustained-release preparation. Still more preferably, the matrix type sustained-release preparation according to the present invention comprises (a) a basic drug or a salt thereof, (b) 15 to 50% by weight of an enteric polymer and (c) 12 to 60% by weight of a water-soluble sugar alcohol, based on 100% by weight of the matrix type sustained-release preparation.

Alternatively, the matrix type sustained-release preparation according to the present invention comprises, for example, (a) a basic drug or a salt thereof, (b) 5 to 90% by weight of an enteric polymer: (c) 3 to 70% by weight of a water-soluble sugar alcohol and (d) 1 to 90% by weight of a water-insoluble polymer, based on 100% by weight of the matrix type sustained-release preparation. Preferably, the matrix type sustained-release preparation according to the present invention comprises, for example, (a) a basic drug or a salt thereof, (b) 8 to 70% by weight of an enteric polymer, (c) 5 to 60% by weight of a water-soluble sugar alcohol and (d) 3 to 70% by weight of a water-insoluble polymer, based on 100% by weight of the matrix type sustained-release preparation. More preferably, the matrix type sustained-release preparation according to the present invention comprises, for example, (a) a basic drug or a salt thereof, (b) 10 to 60% by weight of an enteric polymer, (c) 10 to 60% by weight of a water-soluble sugar alcohol and (d) 5 to 50% by weight of a water-insoluble polymer, based on 100% by weight of the matrix type sustained-release preparation. Still more preferably, the matrix type sustained-release preparation according to the present invention comprises, for example, (a) a basic drug or a salt thereof, (b) 15 to 50% by weight of an enteric polymer, (c) 12 to 60% by weight of a water-soluble sugar alcohol and (d) 5 to 35% by weight of a water-insoluble polymer, based on 100% by weight of the matrix type sustained-release preparation.

According to another aspect of the present invention, there is also provided a method for manufacturing a matrix type sustained-release preparation, comprising the steps of: mixing a basic drug or a salt thereof which has higher solubility in a 0.1 N hydrochloric acid solution and a 50 mM phosphate buffer, pH 6.0 than in a neutral aqueous solution, pH 8.0, with at least one enteric polymer; and compression-molding the mixture obtained in the mixing step. The present invention also provides another method for manufacturing a matrix type sustained-release preparation, comprising the steps of: mixing (1) a basic drug or a salt thereof which has solubility in the 0.1 N hydrochloric acid solution and the neutral aqueous solution, pH 6.0 being 1 mg/mL or more, solubility in the basic aqueous solution, pH 8.0 being 0.2 mg/mL or less, and which has solubility in the neutral aqueous solution, pH 6.8 being at least twice its solubility in the basic aqueous solution, pH 8.0 and being not more than half its solubility in the neutral aqueous solution, pH 6.0, with (2) at least one enteric polymer; and compression-molding the mixture obtained in the mixing step. In a preferred aspect of the method for manufacturing a matrix type sustained-release preparation according to the present invention, a water-insoluble polymer is also mixed in the mixing step.

According to further another aspect of the present invention, there is also provided a method for manufacturing a matrix type sustained-release preparation, comprising the steps of: mixing a basic drug or a salt thereof which has higher solubility in a 0.1 N hydrochloric acid solution and a 50 mM phosphate buffer, pH 6.0 than in a 50 mM phosphate buffer, pH 8.0, with at least one enteric polymer; and compression-molding the mixture obtained in the mixing step. The present invention also provides another method for manufacturing a matrix type sustained-release preparation, comprising the steps of: mixing (1) a basic drug or a salt thereof which has solubility in the 0.1 N hydrochloric acid solution and the 50 mM phosphate buffer, pH 6.0 being 1 mg/mL or more, solubility in the 50 mM phosphate buffer, pH 8.0 being 0.2 mg/mL or less, and which has solubility in the 50 mM phosphate buffer, pH 6.8 being at least twice its solubility in the 50 mM phosphate buffer, pH 8.0 and being not more than half its solubility in the 50 mM phosphate buffer, pH 6.0, with (2) at least one enteric polymer; and compression-molding the mixture obtained in the mixing step. In a preferred aspect of the method for manufacturing a matrix type sustained-release preparation according to the present invention, a water-insoluble polymer is also mixed in the mixing step.

Further, the present invention provides a method for manufacturing a matrix type sustained-release preparation, comprising the steps of: mixing (A) a basic drug or a salt thereof which has higher solubility in a 0.1 N hydrochloric acid solution and a 50 mM phosphate buffer, pH 6.0 than in a 50 mM phosphate buffer, pH 8.0, the solubility of the basic drug or the salt thereof in the 50 mM phosphate buffer, pH 6.8 being at least twice its solubility in the 50 mM phosphate buffer, pH 8.0 and being not more than half its solubility in the 50 mM phosphate buffer, pH 6.0, with (B) at least one enteric polymer and (C) at least one water-insoluble polymer; and compression-molding the mixture obtained in the mixing step, Furthermore, the present invention provides a method for manufacturing a matrix type sustained-release preparation, comprising the steps of: mixing (1) a basic drug or a salt thereof which has solubility in the 0.1 N hydrochloric acid solution and the 50 mM phosphate buffer, pH 6.0 being 1 mg/mL or more, solubility in the 50 mM phosphate buffer, pH 8.0 being 0.2 mg/mL or less, and which has solubility in the 50 mM phosphate buffer, pH 6.8 being at least twice its solubility in the 50 mM phosphate buffer, pH 8.0 and being not more than half its solubility in the 50 mM phosphate buffer, pH 6.0, with (2) at least one enteric polymer and (3) at least one water-insoluble polymer; and compression-molding the mixture obtained in the mixing step.

According to a preferred aspect of the present invention, the above method for manufacturing the matrix type sustained-release preparation further comprises granulating the mixture obtained in the mixing step prior to the compression-molding step. Alternatively, the basic drug or the salt thereof, the enteric polymer and the water-insoluble polymer can be mixed in the mixing step. Of course, other pharmaceutically acceptable additives can also be mixed in the mixing step. In a more preferred aspect of the present invention, granulating step is a wet granulating step, more preferably the wet granulating step involves use of a water-soluble binder.

In the method for manufacturing a matrix type sustained-release preparation according to the present invention, a water-soluble sugar and / or a water-soluble sugar alcohol can be mixed in the matrix type sustained-release preparation as necessary, and other pharmacologically acceptable additives can also mixed. Mixing and compression-molding are accomplished by the ordinary methods commonly used in the field of the preparation, without particular limitations. The matrix type sustained-release preparation can be manufactured by the direct method of compression-molding using a tabletting machine after the mixing step. The matrix type sustained-release preparation can also be manufactured by the manufacturing method which comprises the steps of: granulating the mixture after mixing and before compression-molding. For example, any granulating method can be used including wet granulation method, dry granulation method, fluidized bed granulation method, wet extrusion method, spray-drying method and the like.

The matrix type sustained-release preparation is not particularly limited as long as it is an oral preparation. For example, tablets, granules, fine granules, capsules and the like can be manufactured in the present invention. Capsules can be packed with one or more tablets, granules or fine granules based on the matrix type sustained-release preparation according to the present invention. For example, hard capsules can be packed with multiple small-diameter mini-tablets based on the matrix type sustained-release preparation, or with the aforementioned granules or fine granules based on the matrix type sustained-release preparation, or with both tablets based on the matrix sustained-release preparation and granules or fine granules based on the matrix type sustained-release preparation. The matrix type sustained-release preparation can also be given a film coating as necessary. It should be noted that the presence or absence of a water-soluble film coating on the matrix sustained-release preparation according to the present invention has very little effect on the dissolution profile of the basic drug or the salt thereof from the matrix type sustained-release preparation.

The present invention also provides a method of reducing pH dependence of dissolution of a basic drug or a salt thereof at a dissolution time of 2 to 3 hours corresponding to gastric emptying time (at the early stage of the dissolution test) by mixing the basic drug or the salt thereof, the solubility of which is higher in the 0.1 N hydrochloric solution and the neutral aqueous solution, pH 6.0 than in the basic aqueous solution, pH 8.0 with at least one enteric polymer, and then compression-molding the mixture. The method for manufacturing the matrix type sustained-release preparation according to the present invention may also comprise adding at least one water-insoluble polymer. That is, the present invention provides a method of reducing pH dependence of dissolution of the basic drug or the salt thereof at a dissolution time of 2 to 3 hours corresponding to gastric emptying time (at the early stage of the dissolution test) by mixing the basic drug or the salt thereof, the solubility of which is higher in the 0.1 N hydrochloric solution and the neutral aqueous solution, pH 6.0 than in the basic aqueous solution, pH 8.0 with at least one enteric polymer and at least one water insoluble polymer, and then compression-molding the mixture.

Alternatively, the present invention provides a method of reducing pH dependence of dissolution of a basic drug or a salt thereof at a dissolution time of 2 to 3 hours corresponding to gastric emptying time (at the early stage of the dissolution test) by mixing the basic drug or the salt thereof, the solubility of which is higher in the 0.1 N hydrochloric solution and the 50 mM phosphate buffer, pH 6.0 than in the 50 mM phosphate buffer, pH 8.0 with at least one enteric polymer, and then compression-molding the mixture. The method for manufacturing the matrix type sustained-release preparation according to the present invention may also comprise adding at least one water-insoluble polymer. That is, the present invention provides a method of reducing pH dependence of dissolution of the basic drug or the salt thereof at a dissolution time of 2 to 3 hours corresponding to gastric emptying time (at the early stage of the dissolution test) by mixing the basic drug or the salt thereof, the solubility of which is higher in the 0.1 N hydrochloric solution and the 50 mM phosphate buffer, pH 6.0 than in the 50 mM phosphate buffer, pH 8.0 with at least one enteric polymer and at least one water insoluble polymer, and then compression-molding the mixture.

In the present invention, there is provided a method for controlling release of a basic drug or a salt thereof with low pH dependence, comprising the steps of:mixing (1) a basic drug or a salt thereof which has solubility in the 0.1 N hydrochloric acid solution and the 50 mM phosphate buffer, pH 6.0 being 1 mg/mL or more, solubility in the 50 mM phosphate buffer, pH 8.0 being 0.2 mg/mL or less, and which has solubility in the 50 mM phosphate buffer, pH 6.8 being at least twice its solubility in the 50 mM phosphate buffer, pH 8.0 and being not more than half its solubility in the 50 mM phosphate buffer, pH 6.0, with (2) at least one enteric polymer and (3) at least one water-insoluble polymer; and compression-molding the mixture obtained in the mixing step.

The matrix type sustained-release preparation according to the present invention can be manufactured by, for example, the following methods. 130 g of donepezil hydrochloride (Eisai Co. Ltd.), 624 g of Ethocel 10FP (ethylcellulose, Dow Chemical Company), 780 g of Eudragit L100-55 (Röhm GmbH & Co. KG) and 988 g of lactose are mixed in a granulator. Wet granulation is accomplished by adding an aqueous solution of 52 g of hydroxypropyl cellulose dissolved in a suitable amount of purified water, and the resulting grains are heat-dried using a tray dryer, and sieved to obtain the desired granule size. After sizing, 1 g of magnesium stearate (Mallinckrodt Baker, Inc.) based on 99 g of the granule is added and mixed, and a rotary tabletting machine can then be used to obtain tablets with 8 mm in diameter containing 10 mg of donepezil hydrochloride in 200 mg of the tablet. A coating machine can also be used to coat these tablets with a water-soluble film containing hydroxypropyl methylcellulose or the like as its main component.

The matrix type sustained release preparation according to the present invention can also be manufactured by, for example, the following methods. 20 g of memantine hydrochloride (Lachema s.r.o. Czech Republic), 48 g of Ethocel 10FP (ethylcellulose, Dow Chemical Company), 60 g of Eudragit L100-55 (Röhm GmbH & Co. KG) and 66 g of lactose are mixed in a granulator. Wet granulation is accomplished by adding an aqueous solution of 4 g of hydroxypropyl cellulose dissolved in a suitable amount of purified water, and the resulting grains are heat-dried using a tray dryer, and sieved to the desired granule size. After sizing, 1 g of magnesium stearate (Mallinckrodt Baker, Inc.) based on 99 g of granule is added and mixed, and a rotary tabletting machine can then be used to obtain tablets with 8 mm in diameter containing 20 mg of memantine hydrochloride based on 200 mg of the granule. A coating machine can also be used to coat these tablets with a water-soluble film containing hydroxypropyl methylcellulose or the like as its main component.

### (Experimental Example 1)

In a matrix type sustained-release preparation according to the present invention, effects of an enteric polymer in the presence of a water-insoluble on dissolution behavior are evaluated in the following.

Matrix type sustained-release preparations were prepared using donepezil hydrochloride according to Comparative Example 1, and Examples 2 and 4 which are given below, and dissolution tests were performed thereon. Note that the matrix type sustained-release preparations were prepared using ethylcellulose as a water-insoluble polymer and Eudragit L100-55 as an enteric polymer. The ratios of ethylcellulose to Eudragit L100-55 in Comparative Example 1, and Examples 2 and 4 were 25% : 0% by weight, 25% : 25% by weight and 25% : 50% by weight, respectively. Further, the dissolution tests were performed in the following two types of test solutions at a paddle frequency of 50 rpm in accordance with the dissolution test methods of the Japanese Pharmacopoeia, Ed. 14. The dissolution tests were carried out by use of a test solution A as an acidic test solution and a rest solution B as a neutral test solution as set forth below.
Test solution A: 0.1 N hydrochloric acid solution
Test solution B: 50 mM phosphate buffer, pH 6.8 (buffer of 50 mM sodium phosphate solution with a pH adjusted with hydrochloric acid to be from pH 6.75 to pH 6.84)

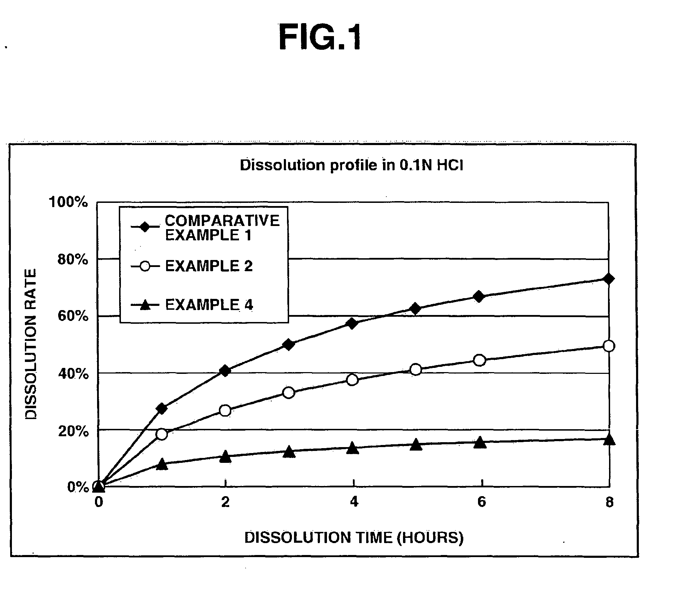

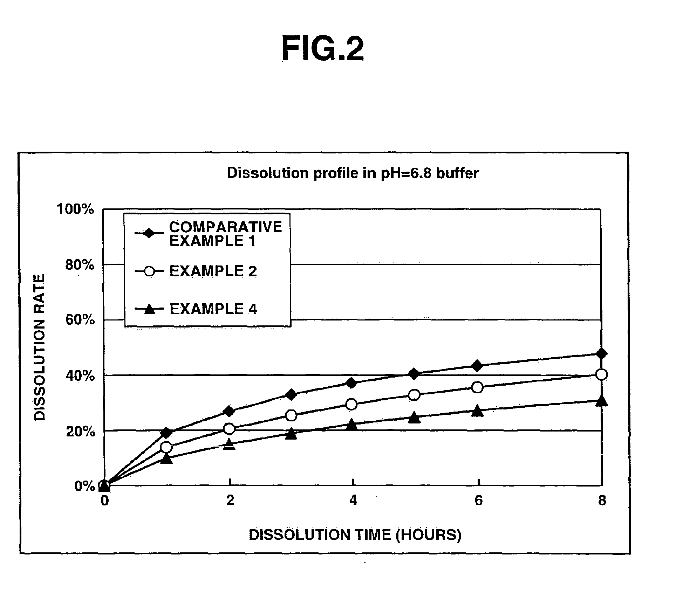

Note that dissolution rate was calculated from concentrations of donepezil hydrochloride in sample solutions collected with dissolution time and analyzed by a spectrophotometric method or HPLC analysis method. The spectrophotometric method was performed under measurement conditions of measurement wavelength at 315 nm, reference wavelength at 650 nm. HPLC analysis method was performed under measurement conditions of measurement column: Inertsil ODS-2 (GL Science), mobile phase: water/acetonitrile/70% aqueous perchloric acid solution = 650/350/1 mixture, and detection wavelength at 271 nm. Comparative results of the dissolution tests are shown in Figures 1 and 2, and results for Comparative Example 1 and Examples 2 and 4 in Tables 1 and 2.

### [Table 1]

**TABLE 1**

| COMPARATIVE EXAMPLE 1 | | | | | EXAMPLE 1 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 27% | 19% | 1.41 | | 1h | 22% | 15% | 1.52 |
| 2h | 41% | 27% | 1.50 | | 2h | 32% | 29% | 1.13 |
| 3h | 50% | 33% | 1.52 | | 3h | 39% | 40% | 0.97 |
| 4h | 57% | 37% | 1.54 | | 4h | 44% | 50% | 0.87 |
| 5h | 63% | 41% | 1.54 | | 5h | 47% | 58% | 0.81 |
| 6h | 67% | 44% | 1.54 | | 6h | 50% | 65% | 0.76 |
| 8h | 73% | 48% | 1.53 | | 8h | 55% | 78% | 0.71 |

### [Table 2]

**TABLE 2**

| EXAMPLE 2 | | | | | EXAMPLE 3 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 18% | 14% | 1.28 | | 1h | 19% | 13% | 1.42 |
| 2h | 27% | 21% | 1.29 | | 2h | 25% | 19% | 1.36 |
| 3h | 33% | 26% | 1.27 | | 3h | 30% | 23% | 1.33 |
| 4h | 37% | 30% | 1.26 | | 4h | 34% | 26% | 1.31 |
| 5h | 41% | 33% | 1.25 | | 5h | 37% | 29% | 1.29 |
| 6h | 44% | 36% | 1.24 | | 6h | 40% | 31% | 1.28 |
| 8h | 50% | 41% | 1.23 | | 8h | 45% | 36% | 1.24 |
| | | | | | | | | |

| EXAMPLE 4 | | | | | EXAMPLE 5 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 8% | 10% | 0.75 | | 1h | 14% | 13% | 1.07 |
| 2h | 11% | 15% | 0.69 | | 2h | 20% | 20% | 0.96 |
| 3h | 12% | 19% | 0.64 | | 3h | 23% | 25% | 0.92 |
| 4h | 14% | 23% | 0.61 | | 4h | 26% | 29% | 0.88 |
| 5h | 15% | 25% | 0.59 | | 5h | 28% | 34% | 0.84 |
| 6h | 16% | 28% | 0.57 | | 6h | 30% | 41% | 0.74 |
| 8h | 17% | 31% | 0.54 | | 8h | 33% | 56% | 0.59 |
| | | | | | | | | |

| EXAMPLE 6 | | | | | | | | |
|---|---|---|---|---|---|---|---|---|
| HOUR | TEST SOLUTION A | TEST SOLUTION B | TEST SOLUTION A/ TEST SOLUTION B | | | | | |
| 1h | 17% | 15% | 1.15 | | | | | |
| 2h | 24% | 29% | 0.83 | | | | | |
| 3h | 29% | 41% | 0.70 | | | | | |
| 4h | 33% | 51% | 0.63 | | | | | |
| 5h | 35% | 60% | 0.59 | | | | | |
| 6h | 38% | 67% | 0.57 | | | | | |
| 8h | 42% | 77% | 0.55 | | | | | |

In Comparative Example 1, and Examples 2 and 4, the weight percentage of Eudragit L100-55 in the tablets varied under a constant 25% by weight of ethylcellulose per 100% by weight of the tablets (0%, 25%, 50% by weight of Eudragit L100-55 per 100% by weight of the tablets, respectively). As shown in Figures 1 and 2, it was confirmed that in dissolution tests using the same test solutions under the same conditions, the greater the content (content percentage) of the enteric polymer (Eudragit L100-55), the slower the dissolution of donepezil hydrochloride from the matrix sustained-release preparation according to the present invention.

In the matrix sustained-release preparation according to the present invention, when both the enteric polymer and the water-insoluble polymer are mixed in the preparation, the greater the amount of the enteric polymer mixed with the water-insoluble polymer, the more the dissolution speed is reduced, thus allowing a long-acting sustained-release preparation to be prepared.

### (Experimental Example 2)

Set out below are the effects of ensuring dissolution with low pH dependence in the matrix type sustained-release preparation, at the same time, of reducing the ratio of dissolution rate of a basic drug or a salt thereof in an acidic test solution to the dissolution rate in a neutral test solution (dissolution rate in the acidic test solution / dissolution rate in the neutral test solution) in a dissolution test, as the dissolution tests proceed.

First, Eudragit L100-55 was used as the enteric polymer and ethylcellulose was used as the water-insoluble polymer in the matrix sustained-release preparation.

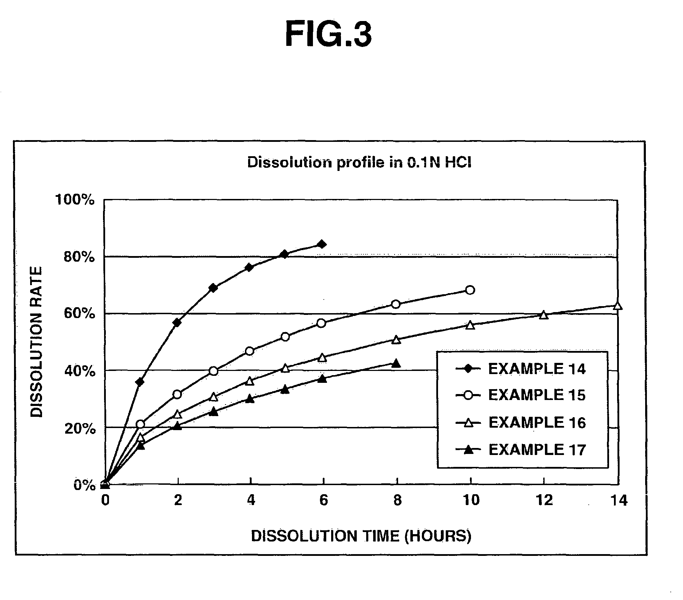

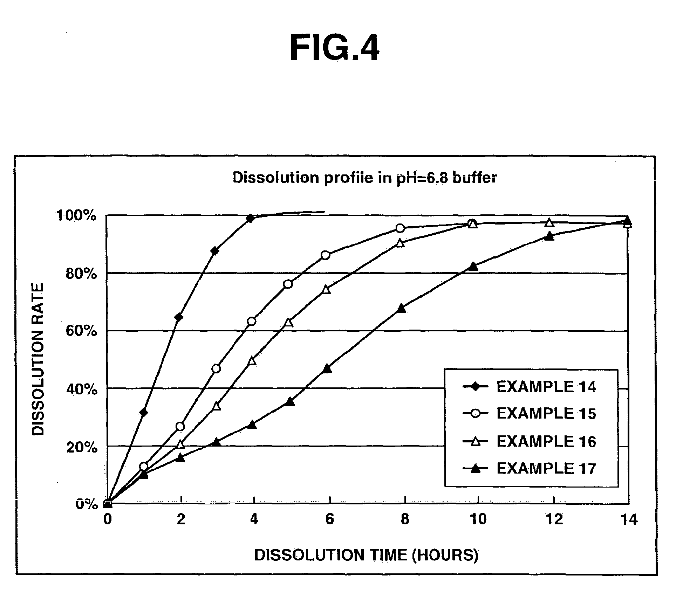

Matrix type sustained release preparations were prepared using donepezil hydrochloride according to Comparative Example 1, and Examples 1 through 11 and 14 through 17 below, and dissolution tests were performed thereon. The dissolution tests were performed to evaluate preparations in which the amounts of donepezil hydrochloride, the enteric polymer and the water-insoluble polymer varied (in Examples 1 to 6), in which the type of diluents varied (in Examples 5 and 7), in which wet granulation was performed using a binder (in Examples 8, 11 and 14 to 17, and 20), in which the type of ethylcellulose varied (in Examples 5, 9 and 10) and in which the scale-up production was carried out (in Examples 11, 14 to 17). Note that a preparation containing donepezil hydrochloride and the water-insoluble polymer as its main components without any enteric polymer was used as Comparative Example 1. The results for Comparative Example 1 and Examples 1, 2 to 6, 7 to 11 and 14 to 17 and 20 are shown in Tables 1, 2, 3 and 4, respectively. Comparative dissolution test results for Examples 14 to 17 are shown in Figures 3 and 4.

### [Table 3]

**TABLE 3**

| EXAMPLE 7 | | | | | EXAMPLE 8 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION | TEST SOLUTION A/ B TEST SOLUTION B |
| 1h | 15% | 16% | 0.98 | | 1h | 17% | 13% | 1.35 |
| 2h | 22% | 24% | 0.92 | | 2h | 25% | 26% | 0.98 |
| 3h | 26% | 30% | 0.86 | | 3h | 32% | 39% | 0.83 |
| 4h | 29% | 36% | 0.81 | | 4h | 37% | 51% | 0.73 |
| 5h | 31% | 45% | 0.68 | | 5h | 42% | 62% | 0.68 |
| 6h | 33% | 57% | 0.59 | | 6h | 45% | 71% | 0.63 |
| 8h | 36% | 69% | 0.53 | | 8h | 51% | 87% | 0.59 |
| | | | | | | | | |

| EXAMPLE 9 | | | | | EXAMPLE 10 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 16% | 15% | 1.06 | | 1h | 16% | 14% | 1.11 |
| 2h | 22% | 28% | 0.80 | | 2h | 22% | 27% | 0.82 |
| 3h | 26% | 39% | 0.68 | | 3h | 26% | 37% | 0.70 |
| 4h | 30% | 49% | 0.61 | | 4h | 30% | 46% | 0.64 |
| 5h | 33% | 57% | 0.57 | | 5h | 33% | 53% | 0.61 |
| 6h | 35% | 64% | 0.55 | | 6h | 35% | 60% | 0.58 |
| 8h | 39% | 76% | 0.51 | | 8h | 39% | 71 % | 0.55 |
| | | | | | | | | |

| EXAMPLE 11 | | | | | | | | |
|---|---|---|---|---|---|---|---|---|
| HOUR | TEST SOLUTION A | TEST SOLUTION B | TEST SOLUTION A/ TEST SOLUTION B | | | | | |
| 1 h | 25% | 16% | 1.56 | | | | | |
| 2h | 37% | 33% | 1.12 | | | | | |
| 3h | 46% | 52% | 0.88 | | | | | |
| 4h | 53% | 69% | 0.77 | | | | | |
| 5h | 59% | 83% | 0.71 | | | | | |
| 6h | 64% | 92% | 0.69 | | | | | |
| 8h | 70% | 99% | 0.71 | | | | | |

### [Table 4]

**TABLE 4**

| EXAMPLE 14 | | | | | EXAMPLE 15 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 36% | 32% | 1.13 | | 1h | 21% | 13% | 1.60 |
| 2h | 56% | 65% | 0.87 | | 2h | 31% | 27% | 1.16 |
| 3h | 69% | 87% | 0.79 | | 3h | 40% | 47% | 0.85 |
| 4h | 76% | 99% | 0.77 | | 4h | 46% | 63% | 0.73 |
| 5h | 81% | 101% | 0.80 | | 5h | 52% | 76% | 0.68 |
| 6h | 84% | 101% | 0.83 | | 6h | 57% | 86% | 0.66 |
| | | | | | 8h | 63% | 95% | 0.66 |
| | | | | | 10h | 68% | 97% | 0.71 |
| | | | | | | | | |

| EXAMPLE 16 | | | | | EXAMPLE 17 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 17% | 11% | 1.49 | | 1h | 14% | 10% | 1.37 |
| 2h | 25% | 21% | 1.17 | | 2h | 21% | 16% | 1.27 |
| 3h | 31% | 34% | 0.90 | | 3h | 26% | 22% | 1.18 |
| 4h | 36% | 50% | 0.72 | | 4h | 30% | 28% | 1.08 |
| 5h | 41% | 63% | 0.64 | | 5h | 34% | 36% | 0.94 |
| 6h | 45% | 75% | 0.60 | | 6h | 37% | 47% | 0.79 |
| 8h | 51% | 90% | 0.56 | | 8h | 43% | 68% | 0.63 |
| 10h | 56% | 97% | 0.58 | | 10h | - | 82% | - |
| 12h | 60% | 97% | 0.61 | | 12h | - | 92% | - |
| 14h | 63% | 97% | 0.65 | | 14h | - | 98% | - |
| | | | | | | | | |

| EXAMPLE 20 | | | | | | | | |
|---|---|---|---|---|---|---|---|---|
| HOUR | TEST SOLUTION A | TEST SOLUTION B | TEST SOLUTION A/ TEST SOLUTION B | | | | | |
| 1h | 32.0 | 16.7 | 1.92 | | | | | |
| 2h | 43.0 | 27.0 | 1.59 | | | | | |
| 3h | 52.0 | 46.0 | 1.13 | | | | | |
| 4h | 58.0 | 65.3 | 0.89 | | | | | |
| 5h | 65.0 | 80.3 | 0.81 | | | | | |
| 6h | 72.0 | 92.3 | 0.78 | | | | | |
| 8h | 79.0 | 103.0 | 0.77 | | | | | |
| 10h | 84.0 | 104.0 | 0.81 | | | | | |
| 12h | 87.0 | 103.3 | 0.84 | | | | | |
| 14h | 90.0 | 103.7 | 0.87 | | | | | |

In the case of the matrix type sustained-release preparation of Comparative Example 1, which contained no the enteric polymer, the ratio of dissolution rate in the acidic test solution to dissolution rate in the neutral test solution (dissolution rate in the acidic test solution / dissolution rate in the neutral test solution) increased slightly from a dissolution time of 1 hour to a dissolution time of 2 to 3 hours at the early stage, and the ratio subsequently remained at about 1.5 with little change thereof at the late stage of dissolution. On the other hand, in the preparations of all examples (Examples 1 to 11, Examples 14 to 17) in which the enteric polymer Eudragit L100-55 was contained, the ratio of the dissolution rate decreased from a dissolution time of 1 hour to a dissolution time of 2 to 3 hours, and continued to decrease gradually as the dissolution test proceeded, until completion of the dissolution test or until a dissolution time at which the dissolution rate in the neutral test solution was 90% or more. The ratio of dissolution rates was from 0.6 to 1.3 at a dissolution time of 3 hours in these cases. That is, by mixing the enteric polymer into the preparation, the preparation according to the present invention can provide a preparation in which the dissolution rate in the acidic test solution is inhibited at the early stage of dissolution (corresponding to the gastric retention period), while reducing pH dependence of the basic drug or the salt thereof, and with which a higher dissolution rate in the neutral test solution relative to the dissolution rate in the acidic test solution can be achieved at the late stage of dissolution (which is thought to correspond to the small intestinal retention stage). The both effects at the early and the late stages of dissolution were confirmed with all preparations in which the amounts of donepezil hydrochloride, enteric polymer and water-insoluble polymer varied (in Examples 1 to 6), in which the type of diluents varied (in Examples 5 and 7), in which wet granulation was achieved with a binder (in Examples 8, 11, 14 to 17, and 20), in which the type of ethylcellulose varied (in Examples 5, 9, 10) and in which the manufacturing scale was altered (in Examples 11, 14 to 17, and 20). In Examples 11 and 14 to 16, in particular, because 90% or more of the drug was released in the 50 mM phosphate buffer, pH 6.8 within 8 hours which is the estimated as the upper limit of large intestinal arrival time in humans *(*Int. J. Pharm. Vol. 53, 1989, 107 to 117), there is little risk of decreased bioavailability due to the sustained release characteristics and it is believed that these preparations are extremely useful.

### (Experimental Example 3)

In this experimental example, types of the enteric and the water-insoluble polymers were evaluated for the matrix type sustained-release preparation. First, set out below are experimental examples of the matrix type sustained-release preparations in which hydroxypropyl methylcellulose acetate succinate was used as the enteric polymer and ethylcellulose as the water-insoluble polymer. The matrix type sustained-release preparations were prepared using donepezil hydrochloride according to Comparative Example 2, and Examples 12 and 13 which are given below, and dissolution tests were performed thereon. Hydroxypropyl methylcellulose acetate succinate (AQOAT LF or AQOAT MF; Shin-Etsu Chemical) was used as the enteric polymer and ethylcellulose was used as the water insoluble polymer in the matrix type sustained-release preparations. Note that the amount of hydroxypropyl methylcellulose acetate succinate as the enteric polymer in the preparations was 50% based on the total weight of the preparation.

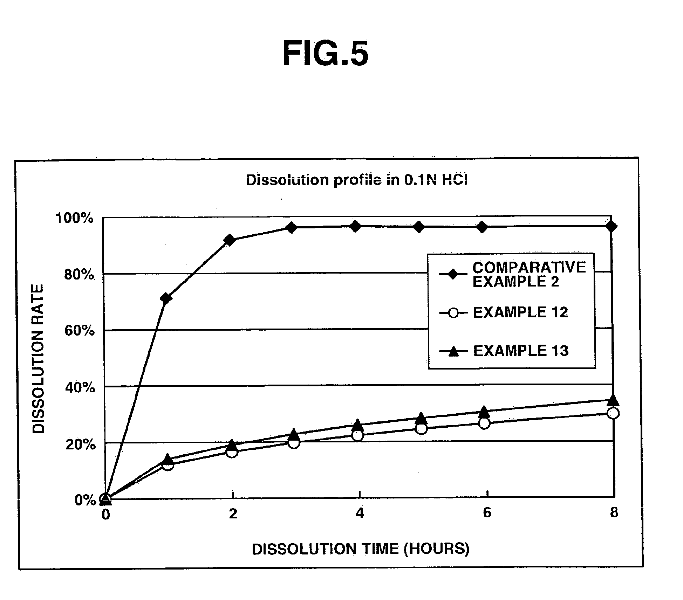

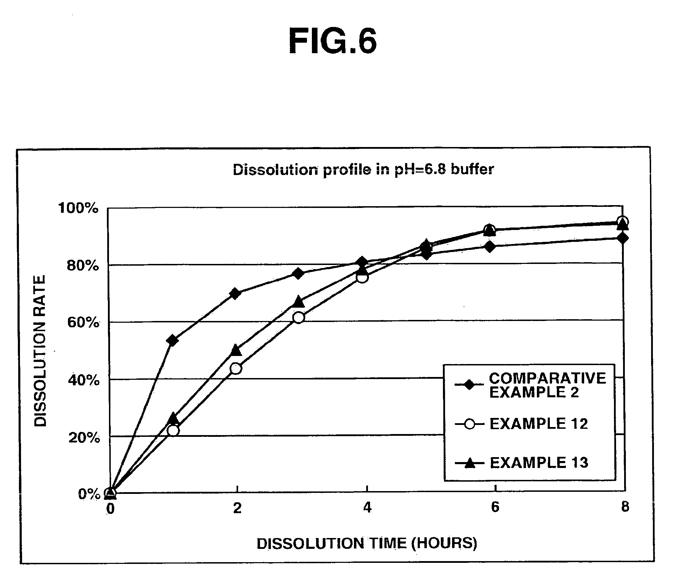

A preparation containing the same amounts of donepezil hydrochloride and the water-insoluble polymer as in Examples 12 and 13 but no enteric polymer was used as Comparative Examples 2. Comparative results of the dissolution tests are shown in Figures 5 and 6, and results for Comparative Example 2, and Examples 12 and 13 are shown in Tables 5.

### [Table 5]

**TABLE 5**

| EXAMPLE 12 | | | | | EXAMPLE 13 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 12% | 22% | 0.55 | | 1h | 14% | 27% | 0.52 |
| 2h | 17% | 44% | 0.38 | | 2h | 19% | 50% | 0.38 |
| 3h | 20% | 61% | 0.32 | | 3h | 23% | 67% | 0.34 |
| 4h | 22% | 75% | 0.30 | | 4h | 26% | 78% | 0.34 |
| 5h | 25% | 85% | 0.29 | | 5h | 29% | 86% | 0.33 |
| 6h | 27% | 91% | 0.29 | | 6h | 31% | 91% | 0.34 |
| 8h | 30% | 94% | 0.32 | | 8h | 35% | 93% | 0.38 |
| | | | | | | | | |

| COMPARATIVE EXAMPLE 2 | | | | | | | | |
|---|---|---|---|---|---|---|---|---|
| HOUR | TEST SOLUTION A | TEST SOLUTION B | TEST SOLUTION A/ TEST SOLUTION B | | | | | |
| 1h | 71% | 53% | 1.34 | | | | | |
| 2h | 92% | 70% | 1.32 | | | | | |
| 3h | 96% | 76% | 1.26 | | | | | |
| 4h | 97% | 80% | 1.20 | | | | | |
| 5h | 97% | 83% | 1.16 | | | | | |
| 6h | 97% | 86% | 1.13 | | | | | |
| 8h | 97% | 88% | 1.09 | | | | | |

As shown in Figures 5 and 6, it was confirmed that dissolution speed of donepezil hydrochloride in an acidic solution was dramatically retarded as a result of addition of 50% enteric polymer (AQOAT LF or AQOAT MF; Shin-Etsu Chemical) to the matrix type sustained-release preparation. In the matrix type sustained-release preparation according to the present invention, the greater the amount of the enteric polymer mixed with the water-insoluble polymer in the preparation containing the enteric polymer and the water-insoluble polymer, the more dissolution speed can be retarded, thus allowing an long-acting sustained-release preparation to be prepared.

With the matrix type sustained-release preparation of Comparative Example 2, which did not contain the enteric polymer, the dissolution rate in the acidic test solution reached 90% at a dissolution time of 2 hours, and the ratio of dissolution rate in the acidic test solution to dissolution rate in the neutral test solution (dissolution rate in the acidic test solution / dissolution rate in the neutral test solution) remained roughly constant at 1.3 at the early stage of dissolution (1 to 3 hours), while in the preparations (Examples 12 and 13) in which 50.0% of hydroxypropyl methylcellulose acetate succinate (AQOAT LF or AQOAT MF; Shin-Etsu Chemical) as the enteric polymer was mixed in the preparation, the ratio of the dissolution rate indicated 0.38 to 0.55, which was lower than that in Comparative Example 2. That is, the mixture of the enteric polymer had the effect of retarding the dissolution rate of the drug in the acidic and neutral test solutions at the early stage of dissolution and in particular of dramatically retarding the dissolution rate of the drug in the acidic test solution, thus bringing the dissolution rates in the two solutions closer to each other and reducing pH dependence. Moreover, at the late stage of dissolution it also had the effect of retarding dissolution in the acidic test solution while increasing dissolution in the neutral test solution. This suggests that the risk of adverse events due to the sustained release characteristics at the early stage of dissolution can be reduced, and the risk of reduced bioavailability can be inhibited in these preparations. Accordingly, it was confirmed that by setting the added amount of hydroxypropyl cellulose acetate succinate (AQOAT LF or AQOAT MF; Shin-Etsu Chemical) to an appropriate value between 0 and 50%, it is possible to design a preparation in which dissolution behavior with the ratio of dissolution rate in the acidic test solution to dissolution rate in the neutral test solution (dissolution rate in the acidic test solution /dissolution rate in the neutral test solution) being near 1 at the early stage of dissolution can be ensured and in which this ratio of dissolution rates can be decreased until the dissolution rate in a neutral solution reaches 90% or more at the late stage of dissolution.

Next, Table 6 shows that effects of a combination of ethylcellulose and Eudragit L100 on dissolution behavior of the preparation. As compared to Comparative Example 1 which contains 25 % of ethylcellulose, it was confirmed in the case of Example 21 which contains 25 % of ethylcellulose and 50 % of Eudragit L100 that a ratio of dissolution rate of the drug or the salt thereof in the acidic test solution to dissolution rate of the drug or the salt thereof in the neutral test solution (dissolution rate in the acidic test solution / dissolution rate in the neutral test solution) decreased with dissolution time.

### [Table 6]

**TABLE 6**

| EXAMPLE 21 | | | | | COMPARATIVE EXAMPLE 1 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1 h | 12% | 8% | 1.50 | | 1 h | 27% | 19% | 1.41 |
| 2h | 19% | 13% | 1.46 | | 2h | 41% | 27% | 1.50 |
| 3h | 25% | 19% | 1.32 | | 3h | 50% | 33% | 1.52 |
| 4h | 29% | 23% | 1.26 | | 4h | 57% | 37% | 1.54 |
| 5h | 32% | 26% | 1.23 | | 5h | 63% | 41% | 1.54 |
| 6h | 35% | 29% | 1.21 | | 6h | 67% | 44% | 1.54 |
| 8h | 39% | 34% | 1.15 | | 8h | 73% | 48% | 1.53 |
| 10h | 43% | 38% | 1.13 | | | | | |
| 12h | 46% | 41% | 1.12 | | | | | |
| 14h | 49% | 44% | 1.11 | | | | | |

Moreover, effects of Eudragit RSPO as the water-insoluble polymer on the dissolution behavior of the preparation were also evaluated in the following: As shown in table 7, Comparative Example 3 which does not contain the enteric polymer but contain Eudragit RSPO exhibited a drug burst behavior and no effect of sustained-release characteristics of the drug. However, it was confirmed in Examples 22 and 23 which contains Eudragit L100 and AQOAT LF, respectively that dissolution time was prolonged in both test solution A and test solution B, and effect of sustained-release characteristics was accomplished by mixing the enteric polymer into the preparation in these Examples. Further, in Examples 22 and 23, a ratio of dissolution rate of the drug or the salt thereof in the acidic test solution to dissolution rate of the drug or the salt thereof in the neutral test solution (dissolution rate in the acidic test solution / dissolution rate in the neutral test solution) exhibited 0.34 and 0.7 at the dissolution time of 3 hour, respectively, and it was confirmed that the above ratio of the dissolution rate of the drug or the salt thereof in the acidic solution to dissolution rate of the drug or the salt thereof in the neutral solution decreased with dissolution time after dissolution time of 3 hour. In particular, Example 23 showed that 90% or more of the drug was released in the 50 mM phosphate buffer, pH 6.8 within 8 hours which is the estimated as the upper limit of large intestinal arrival time in humans and it is believed that the preparation according to Example 23 is extremely useful.

### [Table 7]

**TABLE 7**

| EXAMPLE 22 | | | | | EXAMPLE 23 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 11% | 12% | 0.92 | | 1h | 10% | 21% | 0.48 |
| 2h | 17% | 22% | 0.77 | | 2h | 16% | 41% | 0.39 |
| 3h | 21% | 30% | 0.70 | | 3h | 19% | 56% | 0.34 |
| 4h | 25% | 38% | 0.66 | | 4h | 21% | 71% | 0.30 |
| 5h | 28% | 46% | 0.61 | | 5h | 23% | 82% | 0.28 |
| 6h | 30% | 52% | 0.58 | | 6h | 26% | 91% | 0.29 |
| 8h | 34% | 64% | 0.53 | | 8h | 29% | 105% | 0.28 |
| 10h | 37% | 74% | 0.50 | | 10h | 32% | 108% | 0.30 |
| 12h | 40% | 82% | 0.49 | | 12h | 35% | 109% | 0.32 |
| 14h | 42% | 88% | 0.48 | | 14h | 38% | 109% | 0.35 |
| | | | | | | | | |

| COMPARATIVE EXAMPLE 3 | | | | | | | | |
|---|---|---|---|---|---|---|---|---|
| HOUR | TEST SOLUTION A | TEST SOLUTION | TEST SOLUTION A/ B TEST SOLUTION B | | | | | |
| 1 h | 68% | 88% | 0.77 | | | | | |
| 2h | 94% | 98% | 0.96 | | | | | |
| 3h | 97% | 101 % | 0.96 | | | | | |
| 4h | 97% | 101% | 0.96 | | | | | |
| 5h | 97% | 102% | 0.95 | | | | | |
| 6h | 98% | 102% | 0.96 | | | | | |
| 8h | 97% | 102% | 0.95 | | | | | |
| 10h | 98% | 102% | 0.96 | | | | | |
| 12h | 98% | 102% | 0.96 | | | | | |
| 14h | 98% | 103% | 0.95 | | | | | |

### (Experimental Example 4)

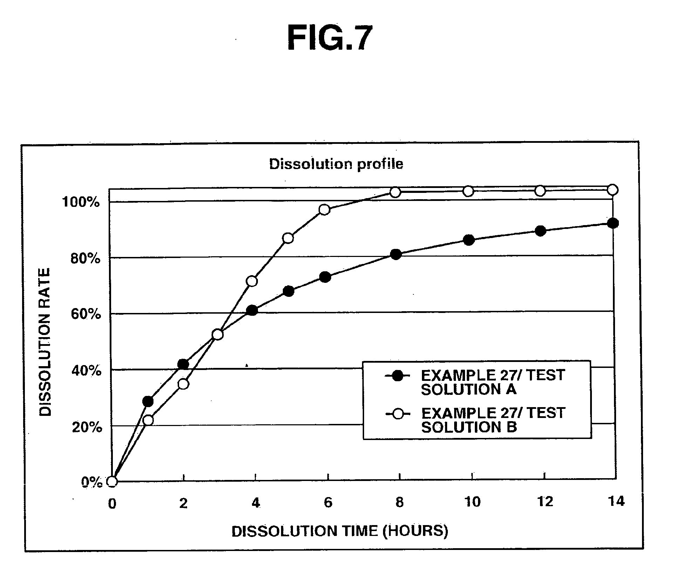

The dissolution tests were carried out using tablets prepared in Examples 27 to 31. The results of the dissolution tests are shown in Table 8 and in Figures 7 to 9. According to the present invention, it is evident that the matrix type sustained-release preparation has the properties of ensuring dissolution with low pH dependence of the basic drug or the salt thereof at the early stage of dissolution and of allowing the ratio of dissolution rate of the basic drug or the salt thereof in the acidic test solution to the dissolution rate of the basic drug or the salt thereof in the neutral test solution (dissolution rate in the acidic test solution / dissolution rate in the neutral test solution) to be decreased at the late stage of dissolution, as the dissolution test proceeds. The these preparations exhibited that 90% or more of the drug was released in the 50 mM phosphate buffer, pH 6.8 within 8 hours which is the estimated as the upper limit of large intestinal arrival time in humans and it is believed that these preparations are extremely useful.

### [Table 8]

**TABLE 8**

| EXAMPLE 27 | | | | | EXAMPLE 28 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 29% | 22% | 1.30 | | 1h | 29% | 15% | 1.87 |
| 2h | 42% | 35% | 1.20 | | 2h | 41% | 31% | 1.32 |
| 3h | 53% | 52% | 1.01 | | 3h | 50% | 47% | 1.06 |
| 4h | 61% | 71% | 0.86 | | 4h | 56% | 61% | 0.93 |
| 5h | 68% | 87% | 0.78 | | 5h | 61 % | 72% | 0.85 |
| 6h | 73% | 97% | 0.75 | | 6h | 66% | 84% | 0.78 |
| 8h | 81% | 103% | 0.78 | | 8h | 74% | 96% | 0.77 |
| 10h | 86% | 104% | 0.83 | | 10h | 79% | 97% | 0.81 |
| 12h | 89% | 104% | 0.86 | | 12h | 82% | 97% | 0.84 |
| 14h | 92% | 104% | 0.88 | | 14h | 84% | 98% | 0.86 |
| | | | | | | | | |

| EXAMPLE 29 | | | | | EXAMPLE 30 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 31% | 20% | 1.51 | | 1h | 29% | 20% | 1.45 |
| 2h | 44% | 34% | 1.29 | | 2h | 43% | 33% | 1.30 |
| 3h | 53% | 47% | 1.14 | | 3h | 54% | 47% | 1.14 |
| 4h | 60% | 59% | 1.02 | | 4h | 62% | 61% | 1.01 |
| 5h | 66% | 70% | 0.94 | | 5h | 70% | 77% | 0.90 |
| 6h | 72% | 81% | 0.89 | | 6h | 76% | 92% | 0.82 |
| 8h | 80% | 96% | 0.83 | | 8h | 85% | 99% | 0.85 |
| 10h | 85% | 97% | 0.87 | | 10h | 90% | 99% | 0.90 |
| 12h | 89% | 97% | 0.91 | | 12h | 94% | 100% | 0.94 |
| 14h | 91% | 97% | 0.94 | | 14h | 96% | 100% | 0.96 |
| | | | | | | | | |

| EXAMPLE 31 | | | | | | | | |
|---|---|---|---|---|---|---|---|---|
| HOUR | TEST SOLUTION A | TEST SOLUTION B | TEST SOLUTION A/ TEST SOLUTION B | | | | | |
| 1h | 29% | 21% | 1.39 | | | | | |
| 2h | 43% | 36% | 1.20 | | | | | |
| 3h | 52% | 51% | 1.03 | | | | | |
| 4h | 61% | 66% | 0.93 | | | | | |
| 5h | 69% | 80% | 0.86 | | | | | |
| 6h | 75% | 91% | 0.82 | | | | | |
| 8h | 83% | 100% | 0.83 | | | | | |
| 10h | 88% | 100% | 0.88 | | | | | |
| 12h | 91% | 100% | 0.91 | | | | | |
| 14h | 94% | 100% | 0.94 | | | | | |

### (Experimental Example 5)

In order to evaluate dissolution behavior of the pharmaceutical composition containing memantine hydrochloride, dissolution tests were carried out using the preparations obtained in the following Examples and Comparative Examples. The dissolution tests were performed in the following two types of test solutions at a paddle frequency of 50 rpm in accordance with the dissolution test methods of the Japanese Pharmacopoeia, Ed. 14. The dissolution tests were carried out by use of a test solution A as an acidic test solution and a test solution B as a neutral test solution as set forth below.
Test solution A: 0.1 N hydrochloric acid solution
Test solution B: 50 mM phosphate buffer, pH 6.8 (buffer of 50 mM sodium phosphate solution with a pH adjusted with hydrochloric acid to be from pH 6.75 to pH 6.84)

Note that dissolution rate was calculated from concentrations of memantine hydrochloride in sample solutions collected with dissolution time and analyzed by a HPLC analysis method after memantine hydrochloride was fluorescent labeled by Fluorescamine. The conditions for fluorescence labeling and HPLC analysis are typically as follows: After sample solutions (1 mL) collected with dissolution time was mixed with borate buffer, pH 9.0 (USP, 9mL), an acetone solution (5 mL) containing Fluorescamine (1.2 mg/mL) was added and stirred enough. Water (10 mL) was also added into the above solution and mixed to obtain a test sample. The test sample was analyzed by HPLC. HPLC analysis was performed under measurement conditions of measurement column: CAPCELL PAK UG120 C18 (Shiseido) or a similar column, column temperature: 40°C, mobile phase: borate buffer, pH 9.0 (USP)/acetonitrile = 60/40 mixture, and detection conditions: fluorescence detector (excitation wavelength / detection wavelength = 391 nm /474nm).

The dissolution tests were performed using tablets obtained in Examples 40 to 42 and Comparative Example 4, in order to evaluate effects of an enteric polymer on the preparations containing memantine hydrochloride and ethylcellulose as the water-insoluble polymer.

Comparative Example 4 which does not contain the enteric polymer but contain ethylcellulose showed that a dissolution rate of memantine hydrochloride was inhibited to be from 30 to 40% at the dissolution time of 1 hour, but a ratio of dissolution rate of the drug or the salt thereof in the acidic test solution to dissolution rate of the drug or the salt thereof in the neutral test solution (dissolution rate in the acidic test solution / dissolution rate in the neutral test solution) was constant without change in the dissolution time. On the other hand, Examples 40 to 42 which contain the enteric polymer showed that dissolution rate of memantine hydrochloride at the early stage of the dissolution was much lower than that in Comparative Example 4, and it was confirmed that dissolution rate of memantine hydrochloride could be inhibited at the early stage of dissolution. Moreover, it was also confirmed in these Examples that the ratio of dissolution rate of the drug or the salt thereof in the acidic test solution to dissolution rate of the drug or the salt thereof in the neutral test solution (dissolution rate in the acidic test solution / dissolution rate in the neutral test solution) decreased with the dissolution time.

### [Table 9]

**TABLE 9**

| EXAMPLE 40 | | | | | EXAMPLE 41 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 10% | 12% | 0.91 | | 1h | 26% | 20% | 1.33 |
| 2h | 14% | 18% | 0.81 | | 2h | 39% | 30% | 1.32 |
| 3h | 18% | 22% | 0.80 | | 3h | 49% | 39% | 1.25 |
| 4h | 19% | 27% | 0.70 | | 4h | 57% | 46% | 1.23 |
| 6h | 22% | 34% | 0.64 | | 6h | 67% | 56% | 1.20 |
| 8h | 23% | 41% | 0.57 | | 8h | 77% | 63% | 1.22 |
| 12h | 28% | 49% | 0.57 | | 12h | 87% | 70% | 1.24 |
| | | | | | | | | |

| EXAMPLE 42 | | | | | COMPARATIVE EXAMPLE 4 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 10% | 21% | 0.49 | | 1 h | 40% | 37% | 1.09 |
| 2h | 14% | 32% | 0.44 | | 2h | 57% | 51% | 1.12 |
| 3h | 18% | 39% | 0.46 | | 3h | 66% | 60% | 1.10 |
| 4h | 20% | 44% | 0.46 | | 4h | 74% | 69% | 1.08 |
| 6h | 25% | 59% | 0.42 | | 6h | 86% | 80% | 1.07 |
| 8h | 29% | 64% | 0.46 | | 8h | 91% | 84% | 1.08 |
| 12h | 35% | 68% | 0.51 | | 12h | 97% | 94% | 1.03 |

Next, dissolution tests were performed using tablets obtained in Example 43 and Comparative Example 5, in order to evaluate effects of an enteric polymer on dissolution behavior of the preparations containing memantine hydrochloride and Eudragit RSPO as the water-insoluble polymer.

Comparative Example 5 which does not contain the enteric polymer but contain Eudragit RSPO showed that a dissolution rate of memantine hydrochloride in the acidic and the neutral test solutions was not less than 90% at 2 hours, a ratio of dissolution rate of the drug or the salt thereof in the acidic test solution to dissolution rate of the drug or the salt thereof in the neutral test solution (dissolution rate in the acidic test solution / dissolution rate in the neutral test solution) was constant without change in the dissolution time. On the other hand, Example 43 which contains the enteric polymer showed that dissolution rate of memantine hydrochloride at the early stage of the dissolution was much lower than that in Comparative Example 5, and it was confirmed that dissolution rate of memantine hydrochloride could be inhibited at the early stage of dissolution. Moreover, it was also confirmed in Example 43 that the ratio of dissolution rate of the drug or the salt thereof in the acidic test solution to dissolution rate of the drug or the salt thereof in the neutral test solution (dissolution rate in the acidic test solution / dissolution rate in the neutral test solution) decreased with the dissolution time.

### [Table 10]

**TABLE 10**

| EXAMPLE 43 | | | | | COMPARATIVE EXAMPLE 5 | | | |
|---|---|---|---|---|---|---|---|---|
| HOUR | TEST SOLUTION A | TEST SOLUTION B | TEST SOLUTION A/ TEST SOLUTION B | | HOUR | TEST SOLUTION A | TEST SOLUTION B | TEST SOLUTION A/ TEST SOLUTION B |
| 1h | 31% | 26% | 1.16 | | 1h | 67% | 87% | 0.77 |
| 2h | 46% | 44% | 1.04 | | 2h | 95% | 90% | 1.06 |
| 3h | 57% | 59% | 0.96 | | 3h | 96% | 94% | 1.02 |
| 4h | 64% | 68% | 0.94 | | 4h | 96% | 95% | 1.01 |
| 6h | 74% | 86% | 0.86 | | 6h | 97% | 94% | 1.03 |
| 8h | 83% | 90% | 0.92 | | 8h | 95% | 93% | 1.02 |
| 12h | 91% | 97% | 0.94 | | 12h | 94% | 93% | 1.01 |

### Example 1

300 mg of donepezil hydrochloride (Eisai Co. Ltd.), 1500 mg of Eudragit L100-55 (Röhm GmbH & Co. KG), 1170 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 1.

### Example 2

300 mg of donepezil hydrochloride (Eisai Co. Ltd.), 750 mg of Ethocel 10FP (ethylcellulose, Dow Chemical Company), 750 mg of Eudragit L100-55 (Röhm GmbH & Co. KG), 1170 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 2.

### Example 3

75 mg of donepezil hydrochloride (Eisai Co. Ltd.), 750 mg of Ethocel 10FP (ethylcellulose, Dow Chemical Company), 750 mg of Eudragit L100-55 (Röhm GmbH & Co. KG), 1395 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 5 mg of donepezil hydrochloride. The results of dissolution test are shown in Table 2.

### Example 4

300 mg of donepezil hydrochloride (Eisai Co. Ltd.), 750 mg of Ethocel 10FP (ethylcellulose, Dow Chemical Company), 1500 mg of Eudragit L100-55 (Röhm GmbH & Co. KG), 420 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 2.

### Example 5

300 mg of donepezil hydrochloride (Eisai Co. Ltd.), 375 mg of Ethocel 10FP (ethylcellulose, Dow Chemical Company), 1500 mg of Eudragit L100-55 (Röhm GmbH & Co. KG), 795 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 2.

### Example 6

300 mg of donepezil hydrochloride (Eisai Co. Ltd.), 183 mg of Ethocel 10FP (ethylcellulose, Dow Chemical Company), 1500 mg of Eudragit L100-55 (Röhm GmbH & Co. KG), 987 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 2.

### Example 7

300 mg of donepezil hydrochloride (Eisai Co. Ltd.), 375 mg of Ethocel 10FP (ethylcellulose, Dow Chemical Company), 1500 mg of Eudragit L100-55 (Röhm GmbH & Co. KG), 795 mg of D-mannitol and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 3.

### Example 8

A suitable amount of purified water was added to and mixed with 300 mg of donepezil hydrochloride (Eisai Co. Ltd.), 375 mg of Ethocel 10FP (ethylcellulose, Dow Chemical Company), 1500 mg of Eudragit L100-55 (Röhm GmbH & Co. KG), 705 mg of lactose and 90 mg of hydroxypropyl cellulose (HPC-L; Nippon Soda Co., Ltd), and the mixture was heat-dried in a thermostatic chamber. 30 mg of magnesium stearate (Mallinckrodt Baker, Inc.) was added to and mixed with the dried granules. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 3.

### Example 9

300 mg of donepezil hydrochloride (Eisai Co. Ltd.), 375 mg of Ethocel 10STD (ethylcellulose, Dow Chemical Company), 1500 mg of Eudragit L100-55 (Röhm GmbH & Co. KG), 795 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 3.

### Example 10

300 mg of donepezil hydrochloride (Eisai Co. Ltd.), 375 mg of Ethocel 100FP (ethylcellulose, Dow Chemical Company), 1500 mg of Eudragit L100-55 (Röhm GmbH & Co. KG), 795 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 3.

### Example 11

70 g of donepezil hydrochloride (Eisai), 336 g of Ethocel 10FP (ethylcellulose, Dow Chemical Company), 364 g of Eudragit L100-55 (Röhm GmbH & Co. KG) and 588 g of lactose were mixed. Wet granulation was carried out by adding an aqueous solution of 28 g of hydroxypropyl cellulose (HPC-L; Nippon Soda Co., Ltd) dissolved in a suitable amount of purified water to this mixture. The resulting granules were heat-dried in a tray dryer, and sieved to obtain the desired granule size. After sizing, 1 g of magnesium stearate (Mallinckrodt Baker, Inc.) based on 99 g of granules was added and mixed. A rotary tabletting machine was used to make the granules into a tablet with 8 mm in diameter containing 10 mg of donepezil hydrochloride in 200 mg of the tablet. The results of dissolution test are shown in Table 3.

### Example 12

300 mg of donepezil hydrochloride (Eisai Co. Ltd.), 375 mg of Ethocel 10FP (ethylcellulose, Dow Chemical Company), 1500 mg of AQOAT LF (hydroxypropyl methylcellulose acetate succinate, Shin-Etsu Chemical), 795 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 5.

### Example 13

300 mg of donepezil hydrochloride (Eisai Co. Ltd.), 375 mg of Ethocel 10FP (ethylcellulose, Dow Chemical Company), 1500 mg of AQOAT MF (hydroxypropyl methylcellulose acetate succinate, Shin-Etsu Chemical), 795 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 5.

### Example 14

130 g of donepezil hydrochloride (Eisai Co. Ltd.), 312 g of Ethocel 10FP (ethylcellulose, Dow Chemical Company), 624 g of Eudragit L100-55 (Röhm GmbH & Co. KG) and 1456 g of lactose were mixed in a granulator. Wet granulation was carried out by adding an aqueous solution of 52 g of hydroxypropyl cellulose (HPC-L; Nippon Soda Co., Ltd) dissolved in a suitable amount of purified water to this mixture. The resulting granules were heat-dried in a tray dryer, and sieved to obtain the desired granule size. After sizing, 1 g of magnesium stearate (Mallinckrodt Baker, Inc.) based on 99 g of granules was added and mixed, and a rotary tabletting machine was used to make a tablet with 8 mm in diameter containing 10 mg of donepezil hydrochloride in 200 mg of the tablet. Opadry yellow (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 4.

### Example 15

130 g of donepezil hydrochloride (Eisai Co. Ltd.), 624 g of Ethocel 10FP (ethylcellulose, Dow Chemical Company), 780 g of Eudragit L100-55 (Röhm GmbH & Co. KG) and 988 g of lactose were mixed in a granulator. Wet granulation was carried out by adding an aqueous solution of 52 g of hydroxypropyl cellulose (HPC-L; Nippon Soda Co., Ltd) dissolved in a suitable amount of purified water to this mixture. The resulting granules were heat-dried in a tray dryer, and sieved to obtain the desired granule size. After sizing, 1 g of magnesium stearate (Mallinckrodt Baker, Inc.) based on 99 g of granules was added and mixed, and a rotary tabletting machine was used to make a tablet with 8 mm in diameter containing 10 mg of donepezil hydrochloride in 200 mg of the tablet. Opadry yellow (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 4.

### Example 16

130 g of donepezil hydrochloride (Eisai Co. Ltd), 780 g of Ethocel 10FP (ethylcellulose, Dow Chemical Company), 858 g of Eudragit L100-55 (Röhm GmbH & Co. KG) and 754 g of lactose were mixed in a granulator. Wet granulation was carried out by adding an aqueous solution of 52 g of hydroxypropyl cellulose (HPC-L; Nippon Soda Co., Ltd) dissolved in a suitable amount of purified water to this mixture. The resulting granules were heat-dried in a tray dryer, and sieved to obtain the desired granule size. After sizing, 1 g of magnesium stearate (Mallinckrodt Baker, Inc.) based on 99 g of granules was added and mixed, and a rotary tabletting machine was used to make a tablet with 8 mm in diameter containing 10 mg of donepezil hydrochloride in 200 mg of the tablet. Opadry yellow (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 4.

### Example 17

130 g of donepezil hydrochloride (Eisai Co. Ltd.), 832 g of Etocel 10FP (ethylcellulose, Dow Chemical Company), 962 g of Eudragit L100-55 (Röhm GmbH & Co. KG) and 598 g of lactose were mixed in a granulator. Wet granulation was carried out by adding an aqueous solution of 52 g of hydroxypropyl cellulose (HPC-L; Nippon Soda Co., Ltd) dissolved in a suitable amount of purified water to the mixture, and the resulting granules were heat-dried using a tray drier, and sieved to obtain the desired granule size. After sizing, 1 g of magnesium stearate (Mallinckrodt Baker, Inc.) based on 99 g of granules was added and mixed, and a rotary tabletting machine was used to form a tablet with 8 mm in diameter containing 10 mg of donepezil hydrochloride in 200 mg of the tablet. Opadry yellow (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 4.

### Example 18

12 g of memantine hydrochloride (Lachema s.r.o. Czech Republic), 28.8 g of Ethocel 10FP (ethylcellulose, Dow Chemical Company), 36 g of Eudragit L100-55 (Röhm GmbH & Co. KG) and 39.6 g of lactose were mixed in a granulator. Wet granulation was carried out by adding an aqueous solution of 2.4 g of hydroxypropyl cellulose (HPC-L; Nippon Soda Co., Ltd) dissolved in a suitable amount of purified water to the mixture, and the resulting granules were heat-dried using a tray drier, and sieved to obtain the desired granule size. After sizing, 1 g of magnesium stearate (Mallinckrodt Baker, Inc.) based on 99 g of granules was added and mixed, and a rotary tabletting machine was used to form a tablet with 8 mm in diameter containing 20 mg of memantine hydrochloride in 200 mg of the tablet.

### Example 19

6 g of donepezil hydrochloride (Eisai Co. Ltd.), 12 g of memantine hydrochloride (Lachema s.r.o.), 28.8 g of Ethocel 10FP (ethylcellulose, Dow Chemical Company), 36 g of Eudragit L100-55 (Röhm GmbH & Co. KG) and 45.6 g of lactose were mixed in a granulator. Wet granulation was carried out by adding an aqueous solution of 2.4 g of hydroxypropyl cellulose (HPC-L; Nippon Soda Co., Ltd) dissolved in a suitable amount of purified water to the mixture, and the resulting granules were heat-dried in a tray drier, and sieved to obtain the desired granule size. After sizing, 1 g of magnesium stearate (Mallinckrodt Baker, Inc.) based on 109 g of granules was added and mixed, and a rotary tabletting machine was used for tabletting, resulting in a compression-molded product with 8 mm in diameter containing 10 mg of donepezil hydrochloride and 20 mg of memantine hydrochloride in 220 mg of the product. Opadry yellow (Colorcon Japan Limited) was used to give this compression molded product a water-soluble film coating containing hydroxypropyl methylcellulose as its main component (coating amount: 8 mg/tablet), resulting in film-coated tablet.

The following comparative examples 1 and 2 are given to illustrate the excellent effects of the matrix type sustained-release preparation according to the present invention.

### Comparative Example 1

300 mg of donepezil hydrochloride (Eisai Co. Ltd.), 750 mg of Ethocel 10FP (ethylcellulose, Dow Chemical Company), 1920 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 1.

### Comparative Example 2

300 mg of donepezil hydrochloride (Eisai Co. Ltd.), 375 mg of Ethocel 10FP (ethylcellulose, Dow Chemical Company), 2295 mg of lactose and 30 mg of magnesium stearate (Mallinckrodt Baker, Inc.) were mixed in a mortar. 200 mg of this mixture was taken and made into tablet using an Autograph AG5000A (Shimazu Corporation) to obtain a tablet with 8 mm in diameter containing 20 mg of donepezil hydrochloride. The results of dissolution test are shown in Table 5.

### Example 20

7 g of donepezil hydrochloride (Eisai Co. Ltd.), 37.8 g of Ethocel 10FP (ethylcellulose, Dow Chemical Company), 22.4 g of Eudragit L 00-55 (Röhm GmbH & Co. KG) and 68.18 g of lactose were mixed in a granulator. Wet granulation was carried out by adding an aqueous solution of 4.2 g of hydroxypropyl cellulose (HPC-L; Nippon Soda Co., Ltd) dissolved in a suitable amount of purified water to the mixture, and the resulting granules were heat-dried in a tray drier, and sieved to obtain the desired granule size. After sizing, 0.3 g of magnesium stearate (Mallinckrodt Baker, Inc.) based on 99.7 g of granules was added and mixed, and a single-punch tabletting machine was used to form a tablet with 8 mm in diameter containing 10 mg of donepezil hydrochloride in 200 mg of the tablet. The results of dissolution test are shown in Table 4.

### Examples 21 to 23 and Comparative Example 3

In accordance with component amounts in Table 11, each component was mixed in a mortar. 200 mg of this mixture was taken and made into tablet using an Autograph AG5000A (Shimazu Corporation) to obtain a tablet (tablet weight: 200 mg) with 8 mm in diameter containing 20 mg of donepezil hydrochloride. The results of dissolution test are shown in Tables 6 and 7.

### [Table 11]

**TABLE 11**

| NAME OF COMPONENT | VENDOR | EXAMPLE 21 | EXAMPLE 22 | EXAMPLE 23 | COMPARATIVE EXAMPLE 3 |
|---|---|---|---|---|---|
| Donepezil ·HCl | Eisai | 300 | 300 | 300 | 300 |
| Ethocel 10 FP | Dow Chemical | 750 | - | - | - |
| Eudragit RS PO | Röhm | - | 750 | 750 | 750 |
| Eudragit L100-55 | Röhm | - | 1500 | - | - |
| Eudragit L100 | Röhm | 1500 | - | - | - |
| AQOAT LF | SHIN-ETSU CHEMICAL | - | - | 1500 | - |
| LACTOSE (FlowLac 100) | Meggle | 420 | 435 | 435 | 1935 |
| MAGNESIUM STEARATE | Mallinckrodt | 30 | 15 | 15 | 15 |
| Total | (mg) | 3000 | 3000 | 3000 | 3000 |

### Example 24

3.5 g of donepezil hydrochloride (Eisai Co. Ltd.), 37.8 g of Ethocel 10FP (ethylcellulose, Dow Chemical Company), 22.4 g of Eudragit L100-55 (Röhm GmbH & Co. KG) and 73.5 g of lactose (Pharmatose 200M manufactured by DMV Corporation) were mixed in a granulator. Wet granulation was carried out by adding an aqueous solution of 2.8 g of hydroxypropyl cellulose (HPC-L; Nippon Soda Co., Ltd) dissolved in a suitable amount of purified water to the mixture, and the resulting granules were heat-dried in a tray drier, and sieved to obtain the desired granule size by a power mill. After sizing, 50 mg of calcium stearate (Merck KGaA, Darmstadt, Germany) based on 5000 mg of granules was added and mixed, and an Autograph AG5000A (Shimazu Corporation) was used to make a compression-molded product with 8 mm in diameter containing 5 mg of donepezil hydrochloride in 202 mg of the product, with a compression pressure of 1200 Kgf.

### Example 25

700 g of donepezil hydrochloride (Eisai Co. Ltd.), 2700 g of Ethocel 10FP (ethylcellulose, Dow Chemical Company), 2100 g of Eudragit L100-55 (Röhm GmbH & Co. KG) and 4250 g of lactose were mixed in a granulator. Wet granulation was carried out by adding an aqueous solution of 220 g of hydroxypropyl cellulose (HPC-L; Nippon Soda Co., Ltd) dissolved in a suitable amount of purified water to the mixture, and the resulting granules were heat-dried in a fluidized-bed drier, and sieved to obtain the desired granule size. After sizing, 0.3 g of magnesium stearate (Mallinckrodt Baker, Inc.) based on 99.7 g of granules was added and mixed, and a rotary tabletting machine was used to form a tablet with 8 mm in diameter containing 14 mg of donepezil hydrochloride in 200 mg of the tablet. Opadry purple (Colorcon Japan Limited) was used to give the resulting tablet a water-soluble film coating containing hydroxypropyl methylcellulose as its main component (coating amount: 8 mg/tablet), resulting in film-coated tablet.

### Example 26

700 g of donepezil hydrochloride (Eisai Co. Ltd.), 2700 g of Ethocel 10FP (ethylcellulose, Dow Chemical Company), 1900 g of Eudragit L100-55 (Röhm GmbH & Co. KG) and 4450 g of lactose were mixed in a granulator. Wet granulation was carried out by adding an aqueous solution of 220 g of hydroxypropyl cellulose (HPC-L; Nippon Soda Co., Ltd) dissolved in a suitable amount of purified water to the mixture, and the resulting granules were heat-dried in a fluidized-bed drier, and sieved to obtain the desired granule size. After sizing, 0.3 g of magnesium stearate (Mallinckrodt Baker, Inc.) based on 99.7 g of granules was added and mixed, and a rotary tabletting machine was used to form a tablet with 8 mm in diameter containing 14 mg of donepezil hydrochloride in 200 mg of the tablet. Opadry purple (Colorcon Japan Limited) was used to give the resulting tablet a water-soluble film coating containing hydroxypropyl methylcellulose as its main component (coating amount: 8 mg/tablet), resulting in film-coated tablet.

### Example 27

700 g of donepezil hydrochloride (Eisai Co. Ltd.), 2700 g of Ethocel 10FP (ethylcellulose, Dow Chemical Company), 1900 g of Eudragit L100-55 (Röhm GmbH & Co. KG) and 4420 g of lactose were mixed in a granulator. Wet granulation was carried out by adding an aqueous solution of 250 g of hydroxypropyl cellulose (HPC-L; Nippon Soda Co., Ltd) dissolved in a suitable amount of purified water to the mixture, and the resulting granules were heat-dried in a fluidized-bed drier, and sieved to obtain the desired granule size. After sizing, 0.3 g of magnesium stearate (Mallinckrodt Baker, Inc.) based on 99.7 g of granules was added and mixed, and a rotary tabletting machine was used to form a tablet with 8 mm in diameter containing 14 mg of donepezil hydrochloride in 200 mg of the tablet. Opadry purple (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 8 and Figure 7.

### Example 28

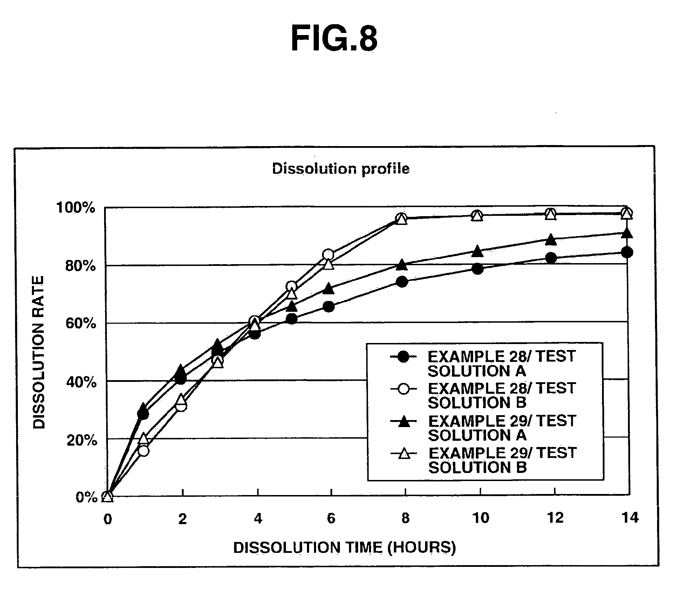

1050 g of donepezil hydrochloride (Eisai Co. Ltd.), 3780 g of Ethocel 10FP (ethylcellulose, Dow Chemical Company), 2240 g of Eudragit L100-55 (Röhm GmbH & Co. KG) and 6538 g of lactose were mixed in a granulator. Wet granulation was carried out by adding an aqueous solution of 350 g of hydroxypropyl cellulose (HPC-L; Nippon Soda Co., Ltd) dissolved in a suitable amount of purified water to the mixture, and the resulting granules were heat-dried in a fluidized-bed drier, and sieved to obtain the desired granule size. After sizing, 0.3 g of magnesium stearate (Mallinckrodt Baker, Inc.) based on 99.7 g of granules was added and mixed, and a rotary tabletting machine was used to form a tablet with 8 mm in diameter containing 15 mg of donepezil hydrochloride in 200 mg of the tablet. Opadry purple (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 8 and Figure 8.

### Example 29

1400 g of donepezil hydrochloride (Eisai Co. Ltd.), 3500 g of Ethocel 10FP (ethylcellulose, Dow Chemical Company), 2520 g of Eudragit L100-55 (Röhm GmbH & Co. KG) and 6118 g of lactose were mixed in a granulator. Wet granulation was carried out by adding an aqueous solution of 420 g of hydroxypropyl cellulose (HPC-L; Nippon Soda Co., Ltd) dissolved in a suitable amount of purified water to the mixture, and the resulting granules were heat-dried in a fluidized-bed drier, and sieved to obtain the desired granule size. After sizing, 0.3 g of magnesium stearate (Mallinckrodt Baker, Inc.) based on 99.7 g of granules was added and mixed, and a rotary tabletting machine was used to form a tablet with 8 mm in diameter containing 20 mg of donepezil hydrochloride in 200 mg of the tablet. Opadry red (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 8 and Figure 8.

### Example 30

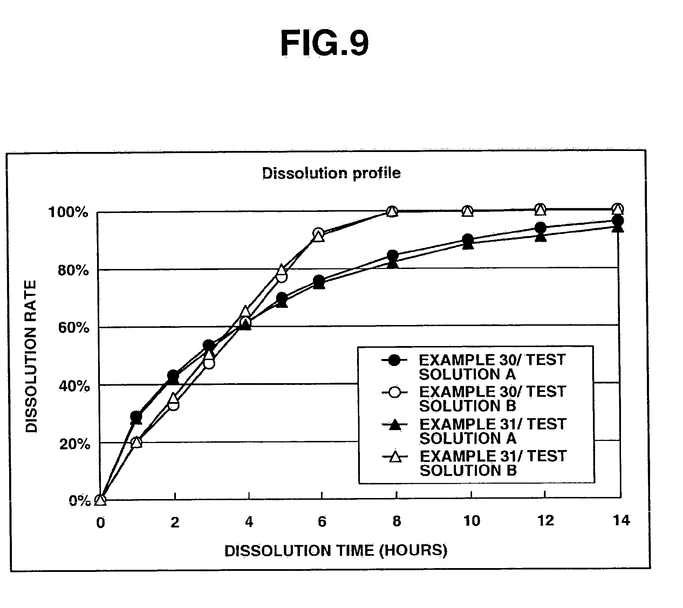

1150 g of donepezil hydrochloride (Eisai Co. Ltd.), 2500 g of Ethocel 10FP (ethylcellulose, Dow Chemical Company), 1800 g of Eudragit L100-55 (Röhm GmbH & Co. KG) and 4220 g of lactose were mixed in a granulator. Wet granulation was carried out by adding an aqueous solution of 300 g of hydroxypropyl cellulose (HPC-L; Nippon Soda Co., Ltd) dissolved in a suitable amount of purified water to the mixture, and the resulting granules were heat-dried in a fluidized-bed drier, and sieved to obtain the desired granule size. After sizing, 0.3 g of magnesium stearate (Mallinckrodt Baker, Inc.) based on 99.7 g of granules was added and mixed, and a rotary tabletting machine was used to form a tablet with 8 mm in diameter containing 23 mg of donepezil hydrochloride in 200 mg of the tablet. Opadry red (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 8 and Figure 9.

### Example 31

1150 g of donepezil hydrochloride (Eisai Co. Ltd.), 2200 g of Ethocel 10FP (ethylcellulose, Dow Chemical Company), 2100 g of Eudragit L100-55 (Röhm GmbH & Co. KG) and 4220 g of lactose were mixed in a granulator. Wet granulation was carried out by adding an aqueous solution of 300 g of hydroxypropyl cellulose (HPC-L; Nippon Soda Co., Ltd) dissolved in a suitable amount of purified water to the mixture, and the resulting granules were heat-dried in a fluidized-bed drier, and sieved to obtain the desired granule size. After sizing, 0.3 g of magnesium stearate (Mallinckrodt Baker, Inc.) based on 99.7 g of granules was added and mixed, and a rotary tabletting machine was used to form a tablet with 8 mm in diameter containing 23 mg of donepezil hydrochloride in 200 mg of the tablet. Opadry red (Colorcon Japan Limited) was used to give the resulting tablet a water-soluble film coating containing hydroxypropyl methylcellulose as its main component (coating amount: 8 mg/tablet), resulting in film-coated tablet. The results of dissolution test are shown in Table 8 and Figure 9.

### Examples 32 to 38

The film-coated tablets shown in Table 12 can be prepared according to the methods described above. Table 12 shows amounts (mg) of each component in one film-coated tablet.

### [Table 12]

**TABLE 12**

| NAME OF COMPONENT | VENDOR | EXAMPLE 32 | EXAMPLE 33 | EXAMPLE 34 | EXAMPLE 35 | EXAMPLE 36 | EXAMPLE 37 | EXAMPLE 38 |
|---|---|---|---|---|---|---|---|---|
| Donopezil·HCl | Eisai | 12 | 12 | 14 | 18 | 18 | 30 | 30 |
| Ethocel 10 FP | Dow Chemical | 54 | 54 | 48 | 54 | 44 | 50 | 44 |
| Eudragit L100-55 | Röhm | 32 | 38 | 44 | 38 | 42 | 36 | 42 |
| Lactose (Pharmatose 200M) | DMV International | 97.4 | 91.4 | 88.4 | 84.4 | 90.4 | 77.4 | 77.4 |
| HPC-L | Nippon Soda | 4 | 4 | 5 | 5 | 5 | 6 | 6 |
| Magnesium Stearate | Mallinckrodt | 0.6 | 0.6 | 0.6 | 0.6 | 0.6 | 0.6 | 0.6 |
| tablet total | (mg) | 200 | 200 | 200 | 200 | 200 | 200 | 200 |
| Opadry purple (mg/tablet) | Colorcon Japan | 8 | 8 | 8 | 8 | 8 | 8 | 8 |
| Film-coated total (mg) | | 208 | 208 | 208 | 208 | 208 | 208 | 208 |

### Examples 39 to 44 and Comparative Examples 4 and 5

In accordance with component amounts in Table 13, each component was mixed in a mortar. 200 mg of this mixture was taken and made into tablet using an Autograph AG5000A (Shimazu Corporation) to obtain a tablet (tablet weight: 200 mg) with 8 mm in diameter containing 20 mg of memantine hydrochloride.

### [Table 13]

**TABLE 13**

| NAME OF COMPONENT | VENDOR | EXAMPLE 39 | EXAMPLE 40 | EXAMPLE 41 | EXAMPLE 42 | EXAMPLE 43 | EXAMPLE 44 | COMPARATIVE EXAMPLE 4 | COMPARATIVE EXAMPLE 5 |
|---|---|---|---|---|---|---|---|---|---|
| Memantine·HCl | Lachema s.r.o. | 300 | 300 | 300 | 300 | 300 | 300 | 300 | 300 |
| Ethocel 10 FP | Dow Chemical | - | 750 | 750 | 750 | - | - | 750 | - |
| Eudragit RS PO | Röhm | - | - | - | - | 760 | 760 | - | 760 |
| Eudragit L100-55 | Röhm | 1500 | 1500 | - | - | 1500 | | - | - |
| Eudragit L100 | Röhm | - | - | 1500 | - | - | - | - | - |
| AQQAT LF | SHIN-ETSU CHEMICAL | - | - | - | 1500 | - | 1500 | - | - |
| LACTOSE (FlowLac 100) | Meggle | 1170 | 420 | 420 | 420 | 435 | 435 | 1920 | 1935 |
| MAGNESIUM STEARATE | Mallinckrodt | 30 | 30 | 30 | 30 | 15 | 15 | 30 | 15 |
| Total (mg) | | 3000 | 3000 | 3000 | 3000 | 3000 | 3000 | 3000 | 3000 |

### Industrial Applicability

According to the present invention, in a matrix type sustained-release preparation containing a basic drug or a salt thereof, which has higher solubility in the 0.1 N hydrochloric acid solution and the neutral aqueous solution, pH 6.0 than in the basic aqueous solution, pH 8.0, the pH dependence of dissolution of the drug or the salt thereof at the early stage of dissolution is reduced, and the ratio of the dissolution rate of the drug or the salt thereof in the acidic test solution to the dissolution rate of the drug or the salt thereof in the neutral test solution (dissolution rate in the acidic test solution / dissolution rate in the neutral test solution) decreases with dissolution time as the dissolution test proceeds (the ratio being lower at the late stage than at the early stage of the dissolution test).

## Claims

1. A matrix type sustained-release preparation comprising:
(1) a basic drug or a salt thereof which has higher solubility in a 0.1 N hydrochloric acid solution and a neutral aqueous solution, pH 6.0 than in a basic aqueous solution, pH 8.0; and
(2) at least one enteric polymer.

2. The matrix type sustained-release preparation according to Claim 1, wherein the neutral aqueous solution is a 50 mM phosphate buffer and the basic aqueous solution is a 50 mM phosphate buffer.

3. The matrix type sustained-release preparation according to Claim 1 or 2, wherein in a dissolution test according to the Japanese Pharmacopoeia paddle method for dissolution tests, a ratio of a dissolution rate of the basic drug or the salt thereof in the 0.1 N hydrochloric acid solution to a dissolution rate of the basic drug or the salt thereof in the 50 mM phosphate buffer, pH 6.8 decreases with dissolution time until a dissolution time at which the dissolution rate of the basic drug or the salt thereof in the 50 mM phosphate buffer, pH 6.8 is 90%.

4. The matrix type sustained-release preparation according to any one of Claims 1 to 3, wherein in the dissolution test according to the Japanese Pharmacopoeia paddle method for the dissolution tests, the dissolution rate of the basic drug or the salt thereof in the 0.1 N hydrochloric acid solution is less than 60% at a dissolution time of 1 hour.

5. The matrix type sustained-release preparation according to any one of Claims 1 to 4, wherein in the dissolution test according to the Japanese Pharmacopoeia paddle method for the dissolution tests, the ratio of the dissolution rate of the basic drug or the salt thereof in the 0.1 N hydrochloric acid solution to the dissolution rate of the basic drug or the salt thereof in the 50 mM phosphate buffer, pH 6.8 is from 0.3 to 1.5 at a dissolution time of 3 hours.

6. The matrix type sustained-release preparation according to any one of Claims 1 to 5, wherein in the dissolution test according to the Japanese Pharmacopoeia paddle method for the dissolution tests, the dissolution rate of the basic drug or the salt thereof in the 0.1 N hydrochloric acid solution is less than 60% at a dissolution time of 1 hour, and the ratio of the dissolution rate of the basic drug or the salt thereof in the 0.1 N hydrochloric acid solution to the dissolution rate of the basic drug or the salt thereof in the 50 mM phosphate buffer, pH 6.8 is from 0.3 to 1.5 at a dissolution time of 3 hours.

7. The matrix type sustained-release preparation according to any one of Claims 1 to 6, further comprising a water-insoluble polymer.

8. The matrix type sustained-release preparation according to any one of Claims 1 to 7, further comprising a water-soluble sugar and / or a water-soluble sugar alcohol.

9. The matrix type sustained-release preparation according to any one of Claims 1 to 8, wherein the enteric polymer is at least one selected from the group consisting of methacrylic acid-ethyl acrylate copolymer, methacrylic acid-methyl methacrylate copolymer, hydroxypropyl methylcellulose phthalate and hydroxypropyl methylcellulose acetate succinate.

10. The matrix type sustained-release preparation according to any one of Claims 7 to 9, wherein the water-insoluble polymer is at least one selected from the group consisting of ethylcellulose, aminoalkyl methacrylate copolymer RS and ethyl acrylate-methyl methacrylate copolymer.

11. The matrix type sustained-release preparation according to any one Claims 1 to 10, wherein the enteric polymer is methacrylic acid-ethyl acrylate copolymer, and the water-insoluble polymer is ethylcellulose.

12. The matrix type sustained-release preparation according to any one Claims 1 to 11, wherein an amount of the enteric polymer in the matrix type sustained-release preparation is from 5 to 90% by weight based on 100% by weight of the matrix type sustained-release preparation.

13. The matrix type sustained-release preparation according to any one Claims 7 to 12, wherein a total amount of the water-insoluble polymer and the enteric polymer in the matrix type sustained-release preparation is from 25 to 95% by weight based on 100% by weight of the matrix type sustained-release preparation.

14. The matrix type sustained-release preparation according to any one Claims 8 to 13, wherein a total amount of the water-soluble sugar and / or the water-soluble sugar alcohol is from 3 to 70% by weight based on 100% by weight of the matrix type sustained-release preparation.

15. The matrix type sustained-release preparation according to any one of Claims 1 to 14, wherein the basic drug or the salt thereof is an anti-dementia drug.

16. The matrix type sustained-release preparation according to any one of Claims 1 to 15, wherein the basic drug or the salt thereof is donepezil hydrochloride and / or memantine hydrochloride.

17. The matrix type sustained-release preparation according to any one of Claims 1 to 16, wherein the solubility of the basic drug or the salt thereof in a neutral aqueous solution, pH 6.8 is at least twice its solubility in a basic aqueous solution, pH 8.0 and is not more than half its solubility in a neutral aqueous solution, pH 6.0.

18. The matrix type sustained-release preparation according to any one of Claims 1 to 17, wherein the solubility of the basic drug or the salt thereof in the 50 mM phosphate buffer, pH 6.8 is at least twice its solubility in the 50 mM phosphate buffer, pH 8.0 and is not more than half its solubility in the 50 mM phosphate buffer, pH 6.0.

19. The matrix type sustained-release preparation according to any one of Claims 1 to 17, wherein the solubility of the basic drug or the salt thereof in the 0.1 N hydrochloric acid solution and the 50 mM phosphate buffer, pH 6.0 is 1 mg/mL or more and the solubility of the basic drug or the salt thereof in the 50 mM phosphate buffer, pH 8.0 is 0.2 mg/mL or less.

20. The matrix type sustained-release preparation according to any one of Claims 1 to 17, wherein the solubility of the basic drug or the salt thereof in the 0.1 N hydrochloric acid solution and the 50 mM phosphate buffer, pH 6.0 is 1 mg/mL or more, the solubility of the basic drug or the salt thereof in the 50 mM phosphate buffer, pH 8.0 is 0.2 mg/mL or less, and the solubility of the basic drug or the salt thereof in the 50 mM phosphate buffer, pH 6.8 is at least twice its solubility in the 50 mM phosphate buffer, pH 8.0 and is not more than half its solubility in the 50 mM phosphate buffer, pH 6.0.

21. The matrix type sustained-release preparation according to any one of Claims 1 to 17, comprising:
(1) the basic drug or the salt thereof which has solubility of 1 mg/mL or more in the 0.1 N hydrochloric acid solution and the 50 mM phosphate buffer, pH 6.0, and which has solubility of 0.2 mg/mL or less in the 50 mM phosphate buffer, pH 8.0, and the solubility of the basic drug or the salt thereof in the 50 mM phosphate buffer, pH 6.8 being at least twice its solubility in the 50 mM phosphate buffer, pH 8.0 and being not more than half its solubility in the 50 mM phosphate buffer, pH 6.0;
(2) at least one enteric polymer; and
(3) at least one water-insoluble polymer.

22. The matrix type sustained-release preparation according to any one of Claims 1 to 21, wherein the matrix type sustained-release preparation is a tablet, a granule, a fine granule or a capsule.

23. A method for manufacturing a matrix type sustained-release preparation comprising the steps of:
mixing a basic drug or a salt thereof which has higher solubility in a 0.1 N hydrochloric acid solution and a neutral aqueous solution, pH 6.0 than in a basic aqueous solution, pH 8.0 with at least one enteric polymer; and
compression-molding the mixture obtained in the mixing step.

24. The method for manufacturing the matrix type sustained-release preparation according to Claim 23, wherein the neutral aqueous solution is a 50 mM phosphate buffer and the basic aqueous solution is a 50 mM phosphate buffer.

25. A method for manufacturing a matrix type sustained-release preparation, comprising the steps of:
mixing (1) a basic drug or a salt thereof which has solubility in the 0.1 N hydrochloric acid solution and the 50 mM phosphate buffer, pH 6.0 being 1 mg/mL or more, solubility in the 50 mM phosphate buffer, pH 8.0 being 0.2 mg/mL or less, and which has solubility in the 50 mM phosphate buffer, pH 6.8 being at least twice its solubility in the 50 mM phosphate buffer, pH 8.0 and being not more than half its solubility in the 50 mM phosphate buffer, pH 6.0, with (2) at least one enteric polymer; and
compression-molding the mixture obtained in the mixing step.

26. The method for manufacturing the matrix type sustained-release preparation according to any one of Claims 23 to 25, wherein a water-insoluble polymer is also mixed in the mixing step.

27. A method for manufacturing a matrix type sustained-release preparation, comprising the steps of:
mixing (1) a basic drug or a salt thereof which has solubility in the 0.1 N hydrochloric acid solution and the 50 mM phosphate buffer, pH 6.0 being 1 mg/mL or more, solubility in the 50 mM phosphate buffer, pH 8.0 being 0.2 mg/mL or less, and which has solubility in the 50 mM phosphate buffer, pH 6.8 being at least twice its solubility in the 50 mM phosphate buffer, pH 8.0 and being not more than half its solubility in the 50 mM phosphate buffer, pH 6.0, with (2) at least one enteric polymer and (3) at least one water-insoluble polymer
compression-molding the mixture obtained in the mixing step.

28. A method for manufacturing a matrix type sustained-release preparation, comprising the steps of:
mixing (A) a basic drug or a salt thereof which has higher solubility in a 0.1 N hydrochloric acid solution and a 50 mM phosphate buffer, pH 6.0 than in a 50 mM phosphate buffer, pH 8.0, the solubility of the basic drug or the salt thereof in the 50 mM phosphate buffer, pH 6.8 being at least twice its solubility in the 50 mM phosphate buffer, pH 8.0 and being not more than half its solubility in the 50 mM phosphate buffer, pH 6.0, with (B) at least one enteric polymer and (C) at least one water-insoluble polymer; and
compression-molding the mixture obtained in the mixing step,

29. The method for manufacturing the matrix type sustained-release preparation according to any one of Claims 23 to 28, further comprising granulating the mixture obtained in the mixing step prior to the compression-molding step.

30. The method for manufacturing the matrix type sustained-release preparation according to any one of Claims 23 to 29, wherein the basic drug or the salt thereof is an anti-dementia drug.

31. The method for manufacturing the matrix type sustained-release preparation according to any one of Claims 23 to 30, wherein the basic drug or the salt thereof is donepezil hydrochloride and / or memantine hydrochloride.

32. A method for controlling release of a basic drug or a salt thereof with low pH dependence, comprising the steps of:
mixing (1) a basic drug or a salt thereof which has solubility in the 0.1 N hydrochloric acid solution and the 50 mM phosphate buffer, pH 6.0 being 1 mg/mL or more, solubility in the 50 mM phosphate buffer, pH 8.0 being 0.2 mg/mL or less, and which has solubility in the 50 mM phosphate buffer, pH 6.8 being at least twice its solubility in the 50 mM phosphate buffer, pH 8.0 and being not more than half its solubility in the 50 mM phosphate buffer, pH 6.0, with (2) at least one enteric polymer and (3) at least one water-insoluble polymer; and
compression-molding the mixture obtained in the mixing step.
